# EUROPEAN PATENT APPLICATION

(11) **EP 4 831 286 A1**
(43) Date of publication of application: **28.10.2026**
(21) Application number: 24907518.5
(22) Date of filing: 19.12.2024
(51) Int. Cl.: C12N 15/09, C12N 9/16, C12N 15/55

(54) **TNPB-LIKE RNA GUIDE NUCLEASE COMPLEX**

(30) Priority: 19.12.2023 JP 2023214275
(71) Applicant: National Institute of Advanced Industrial Science and Technology, Chiyoda-ku Tokyo 100-8921 (JP); Inplanta Innovations Inc., Yokohama-shi, Kanagawa 230-0052 (JP); Toppan Holdings Inc., Tokyo 110-0016 (JP)
(72) Inventor: NAKAMURA, Akiyoshi, Tsukuba-shi, Ibaraki 305-8560 (JP); SUGANO, Shigeo, Tsukuba-shi, Ibaraki 305-8560 (JP); YAMAMOTO, Hiroshi, Tsukuba-shi, Ibaraki 305-8560 (JP); MITSUDA, Nobutaka, Tsukuba-shi, Ibaraki 305-8560 (JP); TERAKAWA, Teruhiko, Yokohama-shi, Kanagawa 230-0052 (JP); YANO, Tsubasa, Yokohama-shi, Kanagawa 230-0052 (JP); HASEGAWA, Reika, Yokohama-shi, Kanagawa 230-0052 (JP); ITO, Seiichiro, Tokyo 110-0016 (JP); SHIRAI, Naomi, Tokyo 110-0016 (JP); NINOMIYA, Yuri, Tokyo 110-0016 (JP)
(74) Representative: Reddie & Grose LLP
(86) International application number: PCT/JP2024/045047
(87) International publication number: WO 2025/135134

(57) **Abstract**

An objective is to identify and utilize novel RNA-guided nucleases applicable to genome editing technologies. The inventors have identified a TnpB-like RNA-guided nuclease that functions as an RNA-guided endonuclease complex applicable to genome editing technology. Based on this finding, the inventors provide a TnpB-like RNA-guided nuclease complex comprising the TnpB-like RNA-guided nuclease and a gRNA comprising a guide sequence and a gRNA scaffold sequence.

## Description

### TECHNICAL FIELD

The present invention relates to a novel TnpB-like RNA-guided nuclease complex, a technique for cleaving a target DNA using the complex, a genome editing technique associated with cleavage, and a technique for regulating transcription in a target DNA using the TnpB-like RNA-guided nuclease complex.

### BACKGROUND ART

Genome editing is a technique for introducing mutations into (editing) a targeted portion of the genome of a target organism. Genome editing technology using CRISPR-Cas is based on the discovery of adaptive immune systems in bacteria and archaea against foreign viruses and plasmids, and is a technology that applies Cas, an RNA-guided endonuclease involved in such adaptive immune systems. DNA from foreign viruses and plasmids is incorporated into the CRISPR locus through the action of Cas proteins. A portion of the foreign DNA once incorporated into the CRISPR locus functions as a template for producing crRNA. The produced crRNA forms a complex with tracrRNA, thereby functioning as a guide strand to recruit Cas protein to the foreign DNA complementary to the crRNA, and cleaves the foreign DNA near the PAM sequence, thereby eliminating the foreign DNA and exerting an immune function. Focusing on the function capable of cleaving a predetermined sequence and designing a guide RNA that performs the functions of crRNA and tracrRNA to be complementary to the sequence of a target DNA enables DNA cleavage at a desired position. Therefore, this technique has the advantage of efficiency in that a desired strain can be obtained from an overwhelmingly smaller number of mutants compared to existing mutation introduction methods such as radiation, and has been used for various applications.

In CRISPR/Cas9 and CRISPR/Cas12a, which are typical genome editing techniques, the sequence to be edited is primarily defined by a portion called a spacer sequence of gRNA or crRNA constituting the sequence. A target sequence having a spacer sequence of gRNA or crRNA and usually capable of being arbitrarily changed for 20 to 24 bases is used. In addition to this target sequence, for Cas9 or Cas12a, which are nucleases, to function, a base sequence of about 2 to 4 bases called a PAM sequence needs to be present in a target nucleic acid sequence. Therefore, genome editing is performed by specifically recognizing a base sequence of a total of about 22 to 28 bases including a PAM sequence and a target sequence. A DNA sequence of a cleavage target needs to contain a PAM sequence, but the PAM sequence varies depending on the type of the Cas protein family, and selecting a Cas protein suitable for the sequence of the target DNA enables genome editing for a desired sequence.

Cas proteins, which are RNA-guided endonucleases, are inferred to originate from IscB and TnpB proteins contained in transposons (Non-Patent Document 1: J. Bacteriol. 198, 797-807 (2015).). IscB protein and TnpB protein, which are nucleases contained in transposons, have been reported to function as RNA-guided endonucleases (Non-Patent Document 2: Science 374, 57-65 (2021), Non-Patent Document 3: Nature 599, 692-696 (2021)). In addition, it has been reported that a complex comprising a protein classified as TnpB including a Ruv-C nuclease domain and an ωRNA molecule can be used for genome editing (Patent Document 1: WO 2022/159892), and research on functional analysis of TnpB, identification of TnpB orthologs, and use thereof as a new genome editing tool is being actively conducted (Non-Patent Document 4: The CRISPR Journal. Jun 2023 232-242, Non-Patent Document 5: Nat Biotechnol., 2023), Non-Patent Document 6: Nature 620, 660-668, 2023, Non-Patent Document 7: Science Advances Sep 2023 vol. 9 Issue 39, Non-Patent Document 8: Nucleic Acids Research, gkad1053, 2023, Non-Patent Document 9: Proc Natl Acad Sci USA. 2023 Nov 28;120(48):e2308224120.).

### LIST OF CITATIONS

### Patent Literature

[Patent Literature 1] WO2022/159892A

### Non-Patent Literature

[Non-Patent Literature 1] J. Bacteriol. (2015) 198, 797-807
[Non-Patent Literature 2] Science (2021) 374, 57-65
[Non-Patent Literature 3] Nature (2021) 599, 692-696
[Non-Patent Literature 4] The CRISPR Journal. (2023) vol. 6, No. 3, p.232-242
[Non-Patent Literature 5] Nat Biotechnol., 2023, June 29, p.1-13
[Non-Patent Literature 6] Nature, 2023, 620, 660-668
[Non-Patent Literature 7] Science Advances, Sep 2023, vol. 9 Issue 39, eadlk0171
[Non-Patent Literature 8] Nucleic Acids Research, gkad1053, 2023
[Non-Patent Literature 9] Proc Natl Acad Sci USA. 2023 Nov 28;120(48):e2308224120.
[Non-Patent Literature 10] Int J Mol Sci., 2020 Apr 25;21(9):3038. doi: 10.3390/ijms21093038.

### SUMMARY OF THE INVENTION

### PROBLEM TO BE SOLVED

An object of the present invention is to identify a novel RNA-guided nuclease applicable to genome editing technology and the use thereof.

### MEANS TO SOLVE THE PROBLEM

The present inventors have conducted intensive research with the aim of identifying a new protein that is not annotated as TnpB and that forms an RNA-guided endonuclease (hereinafter also referred to as RGN) complex applicable to genome editing technology. As a result, the inventors have found a TnpB-like RNA-guided nuclease that is not classified as TnpB, and reached the present invention.

Accordingly, the present invention relates to the following:
[1] A TnpB-like RNA-guided nuclease complex comprising:
   (i) a TnpB-like RNA-guided nuclease that has a length of 300 to 700 amino acids and comprises the following sequence:
      Y-K-G-R-T-F-N-K-M-I-N-N-G-S-K-G-Q-Y-N-x-R-S-x-N-x-L-K-W-R-G
      (SEQ ID NO:109)
      [wherein x represents any amino acid]; and
   (ii) a guide RNA consisting of a sequence comprising a guide sequence and a gRNA scaffold sequence.
[2] The TnpB-like RNA-guided nuclease complex according to aspect 1, wherein the TnpB-like RNA-guided nuclease is a protein consisting of an amino acid sequence selected from the group consisting of SEQ ID NOs: 16, 20, 22, 24, 26, 40, 50, 52, 54, and 56.
[3] The TnpB-like RNA-guided nuclease complex according to aspect 1, wherein the TnpB-like RNA-guided nuclease is a mutant protein having a mutation accompanied by an amino acid substitution relative to the protein according to aspect 2, and having RNA-guided nuclease activity.
[4] The TnpB-like RNA-guided nuclease complex according to aspect 3, wherein the mutant protein consists of an amino acid sequence having at least 75% sequence identity to the amino acid sequence of the TnpB-like RNA-guided nuclease complex according to aspect 2.
[5] The TnpB-like RNA-guided nuclease complex according to aspect 3, wherein the mutant protein is a protein having structural homology to the original protein in a RuvC domain, wherein the structural homology is such that, when structural alignment between the original protein and the mutant protein is performed using PyMOL software, a C-alpha root-mean-square deviation (RMSD) between the backbone of the original protein and the backbone of the mutant protein is 1.5 or less and a TM-score is 0.9 or more.
[6] The TnpB-like RNA-guided nuclease complex according to any one of aspects 1 to 5, wherein a TAM sequence of the TnpB-like RNA-guided nuclease is a sequence comprising TCAT.
[7] The TnpB-like RNA-guided nuclease complex according to aspect 6, wherein the TAM sequence comprises TTCAT.
[8] The TnpB-like RNA-guided nuclease complex according to any one of aspects 1 to 7, wherein the TnpB-like RNA-guided nuclease is a TnpB-like RNA-guided nuclease consisting of the amino acid sequence of SEQ ID NO: 22 or a mutant protein thereof, and the gRNA scaffold sequence comprises the sequence of SEQ ID NO: 58 or a sequence having at least 70% identity to said sequence.
[9] The TnpB-like RNA-guided nuclease complex according to aspect 8, wherein the mutant protein has an amino acid substitution at least at one position selected from the group consisting of Q284, R458, K89, S72 and/or S75, R291, S201, L278, and S315.
[10] The TnpB-like RNA-guided nuclease complex according to aspect 9, wherein in the mutant protein:
   when there is an amino acid substitution at position 284, the substitution is Q284R or Q284K;
   when there is an amino acid substitution at position 89, the substitution is K89R;
   when there is an amino acid substitution at position 72 or 75, the substitution is S72H, S72N, S72Q, S72K, S72R, S75H, S75N, S75Q, S75K, or S75R;
   when there is an amino acid substitution at position 291, the substitution is R291E or R291K;
   when there is an amino acid substitution at position 201, the substitution is Q201H or Q201S;
   when there is an amino acid substitution at position 278, the substitution is L278K or L278R; and
   when there is an amino acid substitution at position 315, the substitution is S315A or S135V.
[11] The TnpB-like RNA-guided nuclease complex according to any one of aspects 1 to 10, wherein the TnpB-like RNA-guided nuclease is a TnpB-like RNA-guided nuclease consisting of the amino acid sequence of SEQ ID NO: 22 or a mutant protein thereof, and the gRNA scaffold sequence comprises a nucleic acid consisting of the nucleotide sequence of SEQ ID NO: 117 (gRNA1), SEQ ID NO: 118 (gRNA2), SEQ ID NO: 119 (gRNA3), or a nucleotide sequence having 90% sequence identity to said sequence.
[12] A method for cleaving a target DNA using the TnpB-like RNA-guided nuclease complex according to any one of aspects 1 to 11.
[13] The method for cleaving a target DNA according to aspect 12, comprising introducing into a cell:
   a nucleic acid comprising an expression cassette encoding the TnpB-like RNA-guided nuclease; and
   a nucleic acid comprising an expression cassette encoding the guide RNA.
[14] The method for cleaving a target DNA according to aspect 12, wherein the cleavage method is used as a genome editing method comprising sequence-specific knockout and sequence-specific knock-in.
[15] An inactivating-mutation-containing TnpB-like RNA-guided nuclease complex comprising:
   (i) a TnpB-like RNA-guided nuclease that has an inactivating mutation in the TnpB-like RNA-guided nuclease of the TnpB-like RNA-guided nuclease complex according to any one of aspects 1 to 11, and fused with a transcriptional regulatory domain; and
   (ii) a guide RNA comprising a guide sequence and a gRNA scaffold sequence.
[16] A method for regulating transcription of a target DNA using the inactivating-mutation-containing TnpB-like RNA-guided nuclease complex according to aspect 15.
[17] The method for regulating transcription of a target DNA according to aspect 15, comprising introducing into a cell:
   an expression cassette encoding the inactivating-mutation-containing TnpB-like RNA-guided nuclease; and
   an expression cassette encoding the guide RNA.
[18] A kit for genome editing comprising the TnpB-like RNA-guided nuclease complex according to any one of aspects 1 to 11.
[19] The kit for genome editing according to aspect 18, wherein the guide RNA comprising the gRNA scaffold sequence comprises a guide sequence designed from a cleavage target sequence.
[20] The kit for genome editing according to aspect 18, wherein a TAM sequence of the TnpB-like RNA-guided nuclease is a sequence comprising TCAT.
[21] The kit for genome editing according to any one of aspects 18 to 20, which is used for sequence-specific knockout and sequence-specific knock-in.

### EFFECT OF THE INVENTION

According to the present invention, a target nucleic acid can be cleaved by a novel RNA-guided endonuclease, which can be applied to genome editing technology.

### BRIEF EXPLANATION OF FIGURES

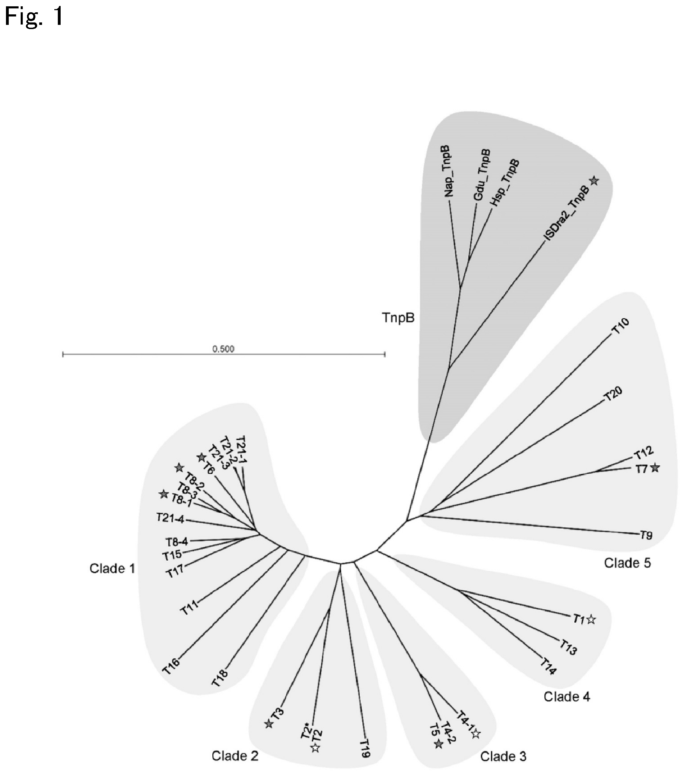

[Figure 1] Figure 1 shows the phylogenetic relationship of T proteins classified into five groups (Clades 1 to 5). The phylogenetic tree was created by the neighbor-joining method using the protein sequences described in Table 1.

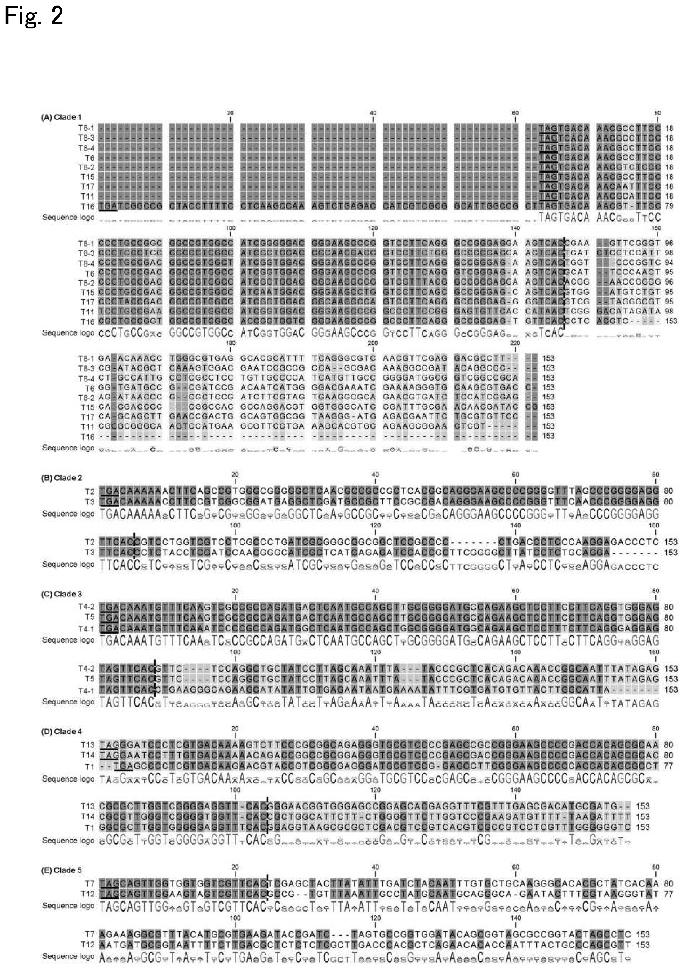

[Figure 2] Figure 2 shows sequences at the 3' end as sequence analysis results for genes encoding TnpB-like RNA-guided nucleases belonging to each clade. Focusing on the boundary at the 3' end of the region with high homology within each clade, such boundary part is determined as a TEM sequence.

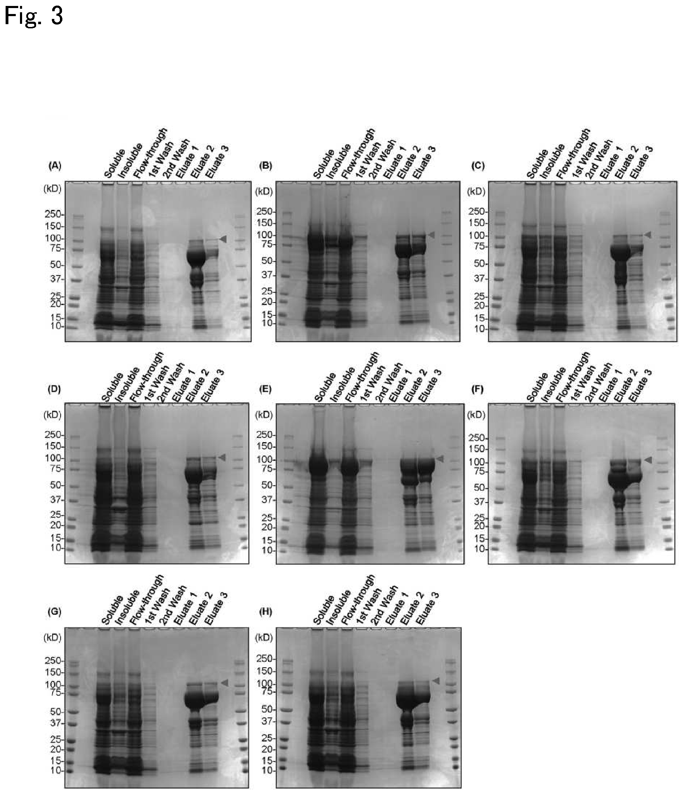

[Figure 3] Figure 3 shows the results of SDS-PAGE separation and Coomassie Blue staining of the TnpB-like RNA-guided nuclease solutions obtained by expressing recombinant His-MBP-TEV fusion TnpB-like RNA-guided nucleases in E. coli followed by column purification (A: T2, B: T3, C: T4, D: T5, E: T6, F: T7, G: T8-1, H: T8-2).

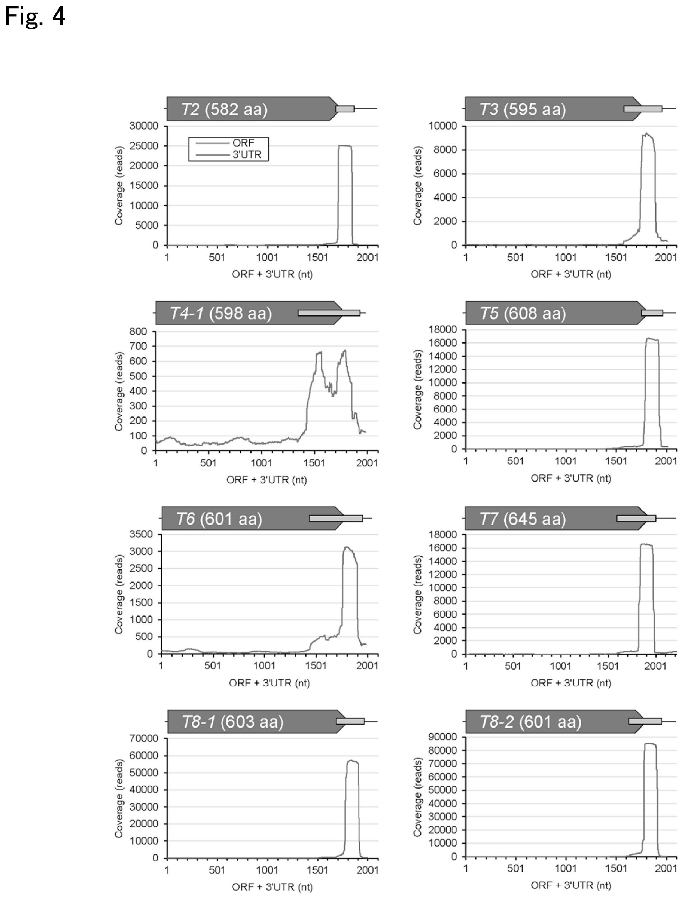

[Figure 4] Figure 4 shows the results of analyzing RNAs that have formed a complex with TnpB-like RNA-guided nuclease by extracting the RNAs from the complexand subjecting the RNAs to next-generation sequencing. It was demonstrated that the RNA forming the complex with TnpB-like RNA-guided nuclease partially overlaps with the open reading frame of the gene for TnpB-like RNA-guided nuclease.

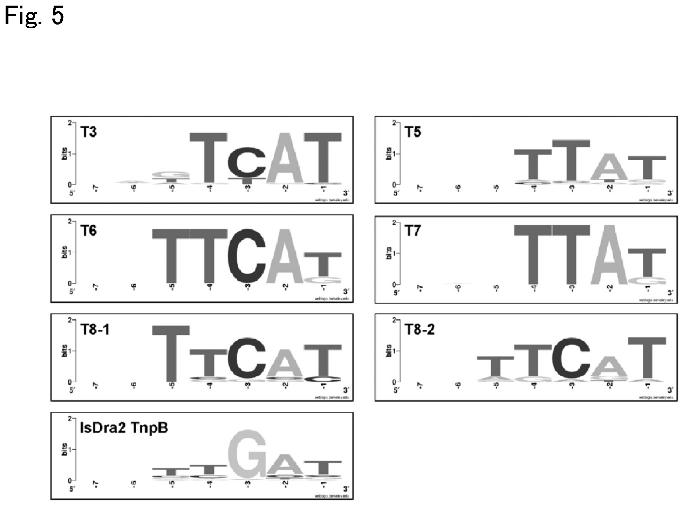

[Figure 5] Figure 5 shows the results of analyzing cleaved plasmids after allowing a complex of TnpB-like RNA-guided nuclease and guide RNA to act on a plasmid library containing random TAMs and target sequences. In the random sequences, plasmids having TCAT (T3), TTAT (T5 and T7), and TTCAT (T6, T8-1, T8-2) were cleaved, and these sequences function as TAM sequences.

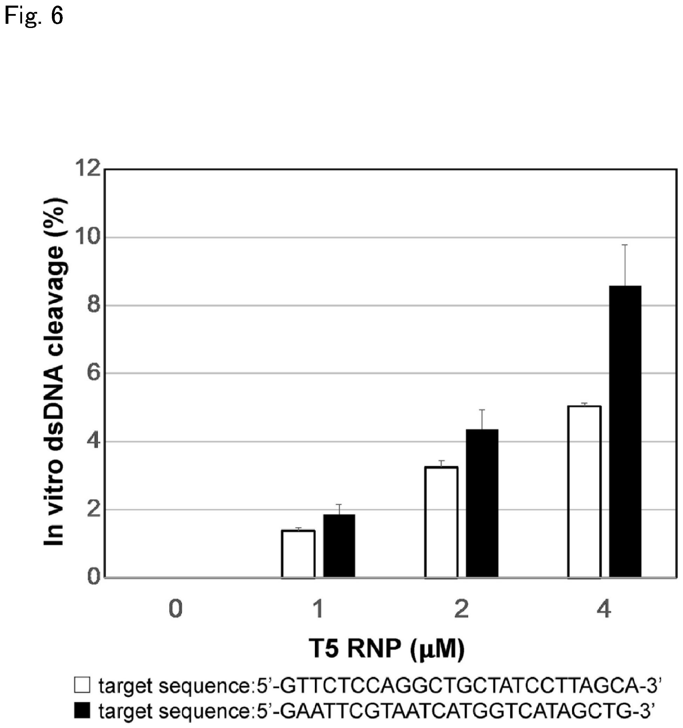

[Figure 6] Figure 6 shows the results of examining cleavage activity by allowing a complex of TnpB-like RNA-guided nuclease and a guide RNA corresponding to a target sequence to act in vitro on double-stranded DNA having different target sequences amplified from a purified plasmid containing a TAM sequence.

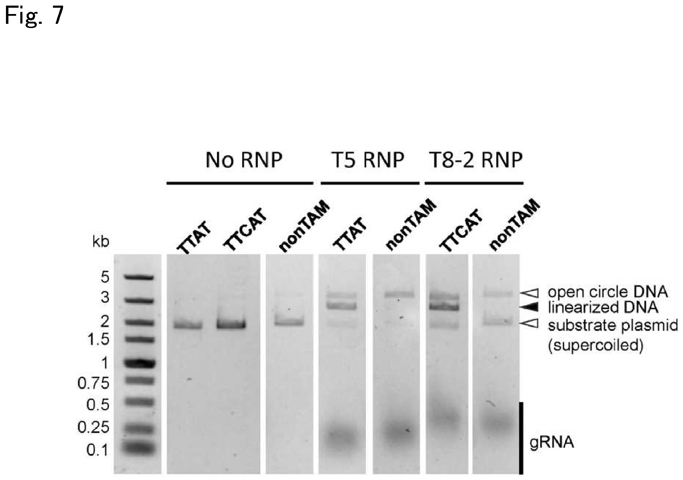

[Figure 7] Figure 7 shows the results of examining cleavage activity by allowing a complex of TnpB-like RNA-guided nuclease (T5 and T8-2) and a guide RNA corresponding to a target sequence to act in vitro on purified plasmids containing different TAM sequences and different target sequences. The types of RNP acted and the TAM sequences used are shown at the top. "No RNP" indicates a negative control.

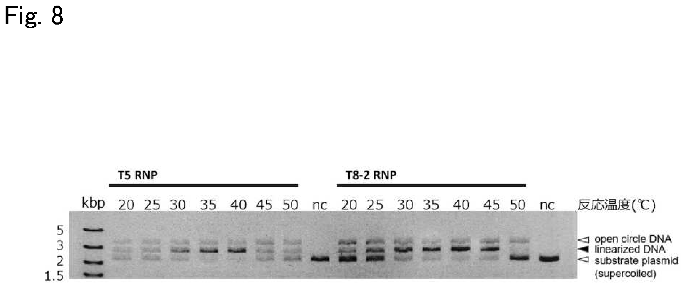

[Figure 8] Figure 8 shows the results of examining cleavage activity when the temperature was varied while acting a complex of TnpB-like RNA-guided nuclease (T5 and T8-2) and a guide RNA corresponding to a target sequence in vitro on purified plasmids containing TAM sequences and target sequences for T5 and T8, respectively.

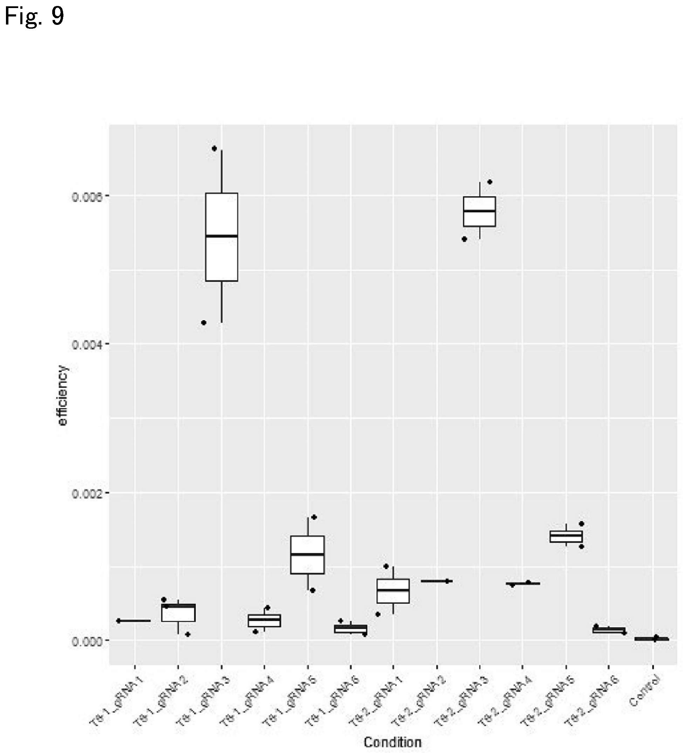

[Figure 9] Figure 9 is a graph showing genome editing efficiency when HEK293FT was transfected with an expression vector encoding T8-1 protein or T8-2 and an expression vector encoding a guide RNA. The vertical axis represents the absolute value of genome editing efficiency, and the horizontal axis represents the conditions when transforming T8-1 gRNA1-5 and T8-2 gRNA1-5, respectively. "Control" shows the genome editing efficiency in the absence of the T8 expression and guide DNA expression plasmids.

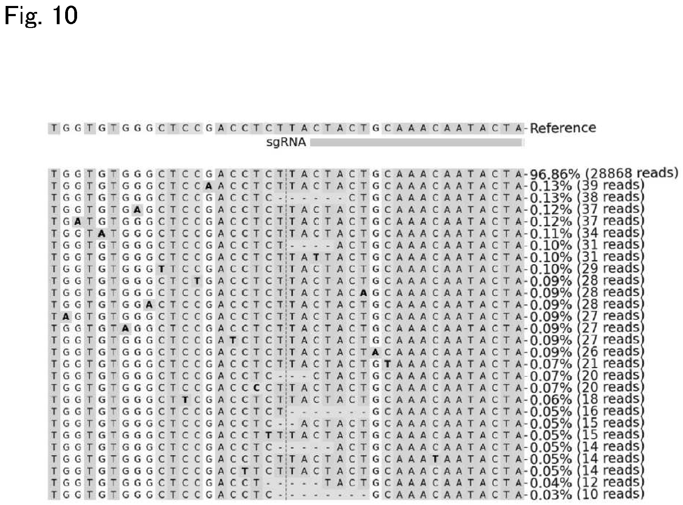

[Figure 10] Figure 10 shows the genomic nucleic acid sequence (5' to the left, 3' to the right) near the cleavage site when HEK293FT was transfected with an expression vector encoding T8-1 protein and an expression vector encoding a guide RNA. sgRNA indicates a target sequence and recognizes the complementary strand of the described DNA. The TAM sequence is present on the 3' side of the target sequence and is not included in this figure. The dashed line indicates the site where the assumed double-strand break occurred.

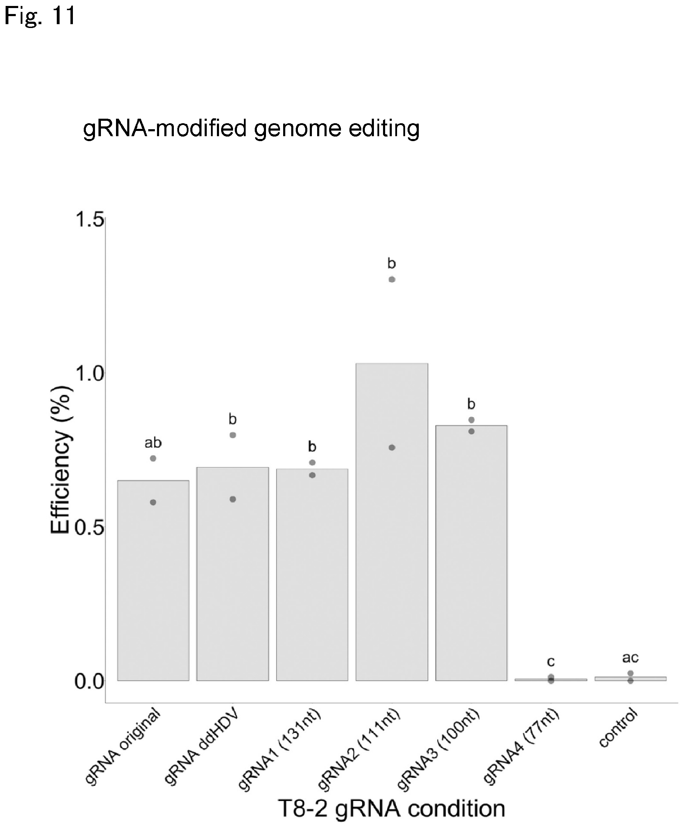

[Figure 11] Figure 11 shows the editing efficiency of modified T8-2 resulting from introduction thereto of gRNA original having a full-length 152 nt T8-2 guide RNA scaffold sequence as a guide RNA, or ddHDV excluding only the HDV sequence, gRNA1 with the T8-2 guide RNA scaffold sequence shortened to 131 nt, gRNA2 with the guide RNA scaffold sequence shortened to 111 nt, gRNA3 shortened to 100 nt, and gRNA4 shortened to 77 nt.

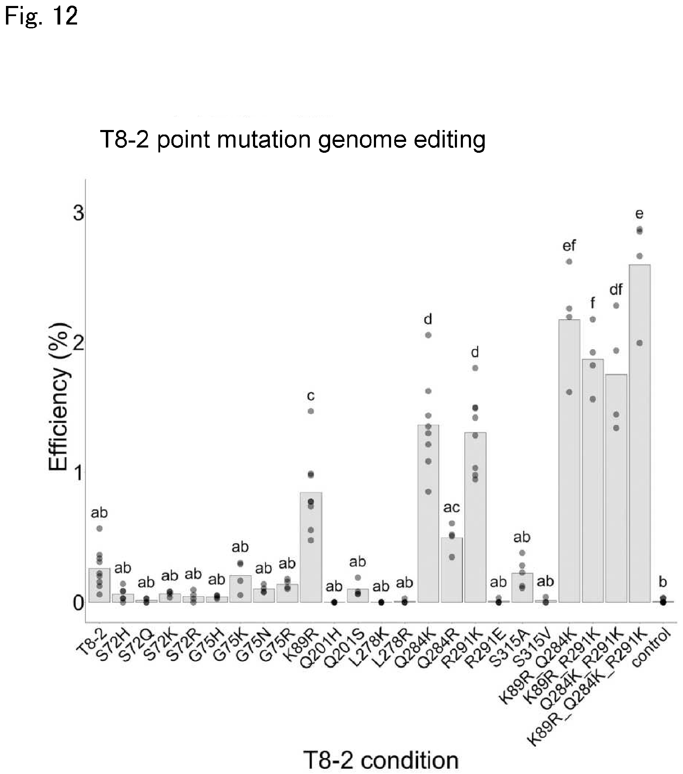

[Figure 12] Figure 12 shows the effect of point mutations in modified T8-2.

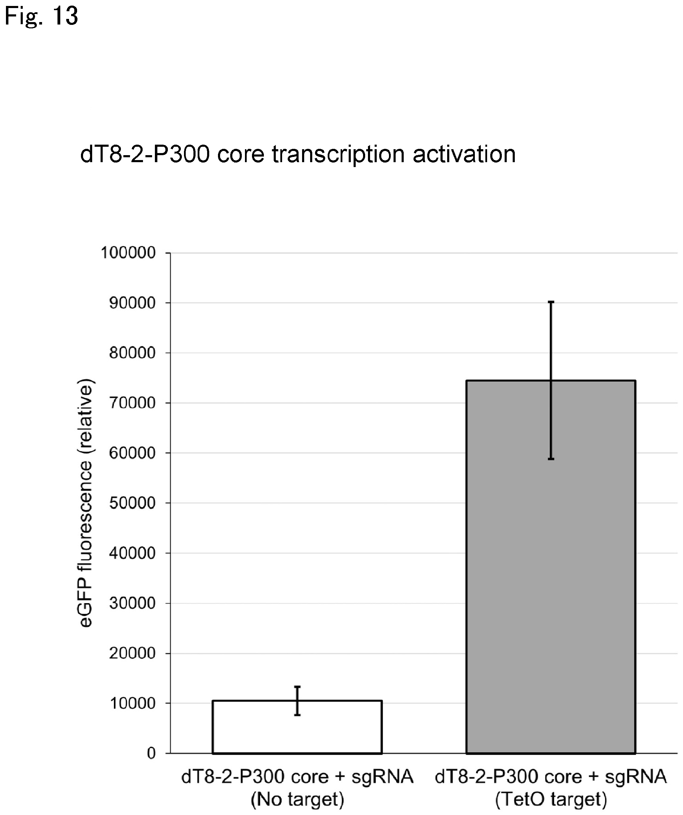

[Figure 13] Figure 13 shows an increase in the production amount of a target protein by an inactivating-mutation-containing TnpB-like RNA-guided nuclease complex comprising a guide RNA and a TnpB-like RNA-guided nuclease in which a p300 core domain is fused to inactivating-mutation-containing T8-2.

### DETAILED EXPLANATION OF INVENTION

One aspect of the present invention relates to a TnpB-like RNA-guided nuclease complex, and also relates to a kit for expressing the complex, a method for cleaving a target DNA using such a TnpB-like RNA-guided nuclease complex, and further relates to a genome editing method.

### [TnpB-like RNA guide nuclease complex]

The TnpB-like RNA-guided nuclease complex according to the present invention comprises: (1) a TnpB-like RNA-guided nuclease; and (2) a gRNA consisting of a sequence comprising a guide sequence and a gRNA scaffold sequence. Such a complex may cleave a predetermined DNA *in vitro*, or may cleave a target DNA at a predetermined position complementary to the sequence of the guide RNA by forming the complex *in vivo*. The complex can be formed by introducing into a cell a vector or messenger RNA comprising expression cassettes encoding (1) the TnpB-like RNA-guided nuclease and (2) the guide RNA, or by introducing the guide RNA itself together with the TnpB-like RNA-guided nuclease. Alternatively, a complex previously formed *in vitro* may be introduced into a cell by a technique such as liposomes or electroporation.

### [TnpB-like RNA guide nuclease]

A TnpB-like RNA-guided nuclease (hereinafter also referred to as a "T protein") forming the TnpB-like RNA-guided nuclease complex includes one or more of the following features:
(i) comprising the following regular expression sequence: 
Y-K-G-R-T-F-[NS]-[KR]-[LM]-x-[AN]-x-G-[AS]-[KR]-[GS]-Q-Y-x(2)-R-[AS]-x-[DN]-
x-L-x-W-x-G
(SEQ ID NO:1)
 (wherein,
   [NS] represents N or S,
   [KR] represents K or R,
   [LM] represents L or S,
   x represents any amino acid,
   [AN] represents A or N,
   [AS] represents A or S,
   [KR] represents K or R,
   [GS] represents G or S,
   x(2) represents any two amino acids,
   [AS] represents A or S, and
   [DN] represents D or N);
(ii) comprising at least one domain selected from the group consisting of RuvCI, bridge helix, RuvCII, RuvCIII, and Wedge;
(iii) having a length of 300 to 700 residues, preferably 500 to 675 residues, and more preferably 550 to 650; and
(iv) having RNA-guided nuclease activity.

The regular expression sequence in the present invention is a sequence conserved among the TnpB-like RNA-guided nucleases of Clades 1 to 4. The regular expression sequence is a 29-amino acid sequence contained in the RuvC domain (more specifically, RuvCIII) of the TnpB-like RNA-guided nuclease. A database search for this regular expression sequence can specifically and comprehensively identify T proteins .

More specifically, the TnpB-like RNA-guided nuclease comprises the following regular expression sequence (i):
Y-K-G-R-T-F-N-K-M-I-N-N-G-S-K-G-Q-Y-N-x-R-S-x-N-x-L-K-W-R-G
(SEQ ID NO:109)
(wherein x independently represents any amino acid), and relates to a TnpB-like RNA-guided nuclease having a length of 300 to 700 amino acids. Such a regular expression sequence refers to TnpB-like RNA-guided nucleases included in Clade 1, excluding T11, T16, and T18.

Specifically, as an example the regular expression sequence may refer to TnpB-like RNA-guided nucleases represented by T6, T8-1 to T8-4, T15, T17, and T21-1 to T21-4.

The bridge helix, RuvCI, RuvCII, and RuvCIII are each thought to be involved in the endonuclease domain, and Cas, IscB, and TnpB also have similar domains. On the other hand, the regular expression sequence in RuvCIII is shared only by TnpB-like RNA-guided nucleases and does not match those of Cas9, Cas12, IscB, and TnpB. The TnpB-like RNA-guided nuclease according to the present invention may comprise all of the domains of the bridge helix, RuvCI, RuvCII, and RuvCIII, from the viewpoint of exerting RNA-guided nuclease activity. Furthermore, the TnpB-like RNA-guided nuclease may be specified by including a common sequence in each domain.

The RNA-guided nuclease activity of the TnpB-like RNA-guided nuclease is such that it recognizes a sequence of 3 to 7 bases, particularly a sequence of 4 bases or 5 bases, called a TAM (Transposon Associated Motif) sequence, and cleaves target sequence DNA downstream thereof. Therefore, the target sequence to which a guide RNA binds is designed to be adjacent to a TAM sequence, and the TnpB-like RNA-guided nuclease cleaves a target nucleic acid containing the target sequence and the TAM sequence. The TAM sequence may differ for each TnpB-like RNA-guided nuclease, but usually the same TAM sequence can be used within the same clade (FIG. 5). In addition, a TAM sequence can be determined by a method well-known in the technical field and can be determined by allowing a complex of a TnpB-like RNA-guided nuclease and a guide RNA for a target sequence to act on a random TAM library in which a random site of several bases, for example, 7 bases, is linked to the target sequence, and confirming cleavage. Examples of typical TAM sequences recognized by the TnpB-like RNA-guided nuclease of each clade are as follows:

**[Table 1]**

| Type of Clade | Typical TAM sequence |
|---|---|
| Clade 1 | TTCAT |
| Clade 2 | TCAT |
| Clade 3 | TTAT |
| Clade 4 | TTAT |
| Clade 5 | TTAT |

TnpB-like RNA-guided nucleases can also be referred to as T proteins (e.g., denoted as T1-T21) and can be phylogenetically classified into Clades 1 to 5. Phylogenetic classification can be performed by methods well known in this technical field. As an example, homologs of TnpB-like RNA-guided nucleases can be searched using NCBI PHI-BLAST with the regular expression of the amino acid sequence (SEQ ID NO: 1) or the regular expression of Clade 1 (SEQ ID NO: 109) as a seed query. From the search result, proteins with sequence homology are further collected using BLASTP software, followed by an alignment such as ClustalW and then a phylogenetic tree can be created from the alignment scores. As another example, the classification can be determined based on a phylogenetic tree analysis of protein sequences performed by the neighbor-joining method, after detecting through NCBI BLASTP against non-redundant protein sequences using the amino acid sequence of T8-2 protein (SEQ ID NO: 22) as a seed query. As still another example, the T-proteins can be classified as a protein family based on the height of RMSD scores using FoldSeek with a partial stereostructure of the RuvC III domain as a query.

The present invention particularly relates to T proteins of Clades 1 to 4.

Proteins included in Clade 1 include T6, T8-1 to T8-4, T11, T15, T16, T17, T18, and T21-1 to T21-4. Among these, the present invention particularly relates to TnpB-like RNA-guided nucleases represented by T6, T8-1 to T8-4, T15, T17, and T21-1 to T21-4, and characterized in that T11, T16, and T18 are excluded.

Proteins included in Clade 2 include T2, T3, and T19. Proteins included in Clade 3 include T4-1, T4-2, and T5.

Proteins included in Clade 4 include T1, T13, and T14.

Proteins included in Clade 5 include T7, T9, T10, T12, and T20.

Phylogenetically, Clade 5 is most closely related to known TnpB, followed by Clade 4, Clade 3, Clade 2, and then Clade 1 in order of increasing distance.

Amino acid sequences and ORF nucleotide sequences of proteins belonging to Clade 1 are as follows:

**[Table 2]**

| Amino acid sequences and nucleotide sequences of Clade 1 | | | | | | |
|---|---|---|---|---|---|---|
| Name | Origin | Genebank ID | Locus tag | Protein ID | ORFnucleic acid sequence | Amino acid sequence |
| T6 | Pararhizobium sp. BTT-229 | JAOYTJ010000003.6 | OIU34_19930 | WP_264264325.1 | SEQ ID NO:17 | SEQ ID NO:16 |
| T8-1 | Agrobacterium salinotolerans ST07.17.032 | JAPZLS010000003.1 | O9X98_08650 | MCZ7861466.1 | SEQ ID NO:21 | SEQ ID NO:20 |
| T8-2 | Agrobacterium salinotolerans ST07.17.032 | JAPZLS010000005.1 | O9X98_14525 | MCZ7862587.1 | SEQ ID NO:23 | SEQ ID NO:22 |
| T8-3 | Agrobacterium salinotolerans ST07.17.032 | JAPZLS010000003.1 | O9X98_10940 | MCZ7861893.1 | SEQ ID NO:25 | SEQ ID NO:24 |
| T8-4 | Agrobacterium salinotolerans ST07.17.032 | JAPZLS010000002.1 | O9X98_04255 | MCZ7860610.1 | SEQ ID NO:27 | SEQ ID NO:26 |
| T11 | Ensifer sp. M14 | NZ_QJNR01000007.1 | BLJAPNOD_RS23510 | WP_115421657.1 | SEQ ID NO:33 | SEQ ID NO:32 |
| T15 | Rhizobium laguerreae strain OyaliA | NZ_JAAXRC010000016.Oyali | HFO56_RS33840 | WP_222046788.1 | SEQ ID NO:41 | SEQ ID NO:40 |
| T16 | Rhizobium sp. S163 soapdenovo | NZ_JAUDSA010000035.163 soapdenov | QTL96_RS24940 | WP_289729600.1 | SEQ ID NO:43 | SEQ ID NO:42 |
| T18 | Mesorhizobium sp. SP-1A | NZ_JAVXZD010000002.1 | QU595_RS12895 | WP_315922544.1 | SEQ ID NO:47 | SEQ ID NO:46 |
| T21-1 | Sinorhizobium meliloti strain 33 NODE 3 | WOHW01000003.1 | GOB57_07870 | MDW9478619.1 | SEQ ID NO:51 | SEQ ID NO:50 |
| T21-2 | Sinorhizobium meliloti strain 33 NODE 3 | WOHW01000010.1 | GOB57_21135 | MDW9481153.1 | SEQ ID NO:53 | SEQ ID NO:52 |
| T21-3 | Sinorhizobium meliloti strain 33 NODE 3 | WOHW01000010.1 | GOB57_21895 | MDW9481302.1 | SEQ ID NO:55 | SEQ ID NO:54 |
| T21-4 | Sinorhizobium meliloti strain 33 NODE_3 | WOHW01000010.1 | GOB57_21515 | MDW9481226.1 | SEQ ID NO:57 | SEQ ID NO:56 |

Amino acid sequences and ORF nucleotide sequences of proteins belonging to Clade 2 are as follows:

**[Table 3]**

| Amino acid sequences and nucleotide sequences of Clade 2 | | | | | | |
|---|---|---|---|---|---|---|
| Name | Origin | Genebank ID | Locus tag | Protein ID | ORFnucleic acid sequence | Amino acid sequence |
| T2 | Gemmobacter caeni strain DSM21823 | NZ_QBKP01000002.2 | C8N34_RS04470 | WP_146177478.1 | SEQ ID NO:5 | SEQ ID NO:4 |
| T2* | Gemmobacter caeni strain CGMCC1.7745 | NZ_VLLH01000003.1 | IQ03_RS26860 | WP_199750653.1 | SEQ ID NO:7 | SEQ ID NO:6 |
| T3 | Cereibacter sphaeroides strain AB170 | NZ_JAIRAT010000143.1 | LAZ39_RS12345 | WP_233830222.1 | SEQ ID NO:9 | SEQ ID NO:8 |
| T19 | Metagenome (MGNify database) | | | MGYP000657443229 | Not disclosed | SEQ ID NO:48 |

Amino acid sequences and ORF nucleotide sequences of proteins belonging to Clade 3 are as follows:

**[Table 4]**

| Amino acid sequences and nucleotide sequences of Clade 3 | | | | | | |
|---|---|---|---|---|---|---|
| Name | Origin | Genebank ID | Locus tag | Protein ID | ORFnucleic acid sequence | Amino acid sequence |
| T4-1 | Candidatus Aquiluna sp. | QCWE01000009.1 | DCO81_07345 | PWL19880.1 | SEQ ID NO:11 | SEQ ID NO:10 |
| T4-2 | Candidatus Aquiluna sp. | QCWE01000013.2 | DCO81_07605 | PWL19831.1 | SEQ ID NO:13 | SEQ ID NO:12 |
| T5 | Altereythrobacter sp. | QCWG01000010.5 | DCO98_12115 | PWL23424.1 | SEQ ID NO:15 | SEQ ID NO:14 |

Amino acid sequences and ORF nucleotide sequences of proteins belonging to Clade 4 are as follows:

**[Table 5]**

| Amino acid sequences and nucleotide sequences of Clade 4 | | | | | | |
|---|---|---|---|---|---|---|
| Name | Origin | Genebank ID | Locus tag | Protein ID | ORFnucleic acid sequence | Amino acid sequence |
| T1 | Methylobacterium sp. V23 | PDHT01000069.1 | CRT23_26605 | POR39947.1 | SEQ ID NO:3 | SEQ ID NO:2 |
| T13 | Microvirga massiliensis isolate MGYG-HGUT-02310 | NZ_CABMGP010000003.0231 | FYC13_RS11430 | WP_046863660.1 | SEQ ID NO:37 | SEQ ID NO:36 |
| T14 | Microvirga massiliensis isolate MGYG-HGUT-02310 | NZ_CABMGP010000011.0231 | FYC13_RS28300 | WP_046867075.1 | SEQ ID NO:39 | SEQ ID NO:38 |

Amino acid sequences and ORF nucleotide sequences of proteins belonging to Clade 5 are as follows:

**[Table 6]**

| Amino aci | | | | | | |
|---|---|---|---|---|---|---|
| Name | Origin | Genebank ID | Locus tag | Protein ID | ORFnucleic acid sequence | Amino acid sequence |
| T7 | Halomonas rituensis TQ8S | NZ_QPIJ01000015.7 | DU506_RS08600 | WP_114486527.1 | SEQ ID NO:19 | SEQ ID NO:18 |
| T9 | Acidobacteriota bacaterium isolate SZAS-15 | JAFDVL010000012.1 | JST84_05285 | MBS1807587.1 | SEQ ID NO:29 | SEQ ID NO:28 |
| T10 | Deltaproteobacteria bacterium NP119 | PCBK01000052.11 | CL920_12435 | MBU49494.1 | SEQ ID NO:31 | SEQ ID NO:30 |
| T12 | Halomonas zhuhanensis strain ZH2S | NZ_WTKP01000005.2 | GMP19_RS08890 | WP_160418682.1 | SEQ ID NO:35 | SEQ ID NO:34 |
| T17 | Rhizobium sp. BK176 | JANUBV010000001.17 | FHT76_001792 | WP_258185500.1 | SEQ ID NO:45 | SEQ ID NO:44 |
| T20 | metagenome (MGNify database) | | | MGYP000135288571 | Not disclosed | SEQ ID NO:48 |

d sequences and nucleotide sequences of Clade 5

| Clade | Sequence | Identity within Clade |
|---|---|---|
| Clade 1 (T8-2) | CCGAGAUGAUCGGGCGCUACCUGUUUCUCAGGCC CAAGAACGGAAUCCAACCAGCGGCCUUGGCCGCCU AGUGACAAACGUCUCCCCCCUGCCGACGGCCGUGG CCAUCGGUGGACGGGAAGCCCGGUCGUUUACGGC CGGGAGGAAGUCAC (SEQ ID NO:58) | 70% |
| Clade 2 (T3) | GGCGGACGGCACCCGGUGGACGCGGACCUGCAU GCCGCGGAGACGCUGGCGCGAUGGCUCUUCCUCA GGCCGAAACCGGCGGGUUCGCCCGCCUUGUGACA AAAACCUUCCGUCGGCGGAUGAGGCUCGAUGCCGC UUCCGCCGACAGGGAAGCCCCGGGUUUCAACCCG GGGAGGUUCAC (SEQ ID NO:59) | 70% |
| Clade 3 (T5) | CCUAAAGAAACAGUCAGUCAGGCCUUUGGUCUGGC UGCUGAGUGACAAAUGUUUCAAGUCGCCGCCAGAU GACUCAAUGCCAGCUUGCGGGGAUGCCAGAAGCUC CUUCCUUCAGGUGGGAGUAGUUCAC (SEQ ID NO:60) | 70% |
| Clade 4 (T13) | GCGGCGCACACGCUGGCGCUGUGGCCUCUGCUGC GGCCGAAGAUGAACGACAGGACGGCGCUGGCCGC CUAGGGAUCCCUCGUGACAAAAGUCUUCCCGCGGC AGAGGGUGCGUCCCCGAGCCGCCGGGAAGCCCCG ACCACAGCGCAACGCGCUUGGUCGGGGAGGUUCA C (SEQ ID NO:61) | 70% |
| Clade 5 (T7) | GUGAAAAAUCCCGGCAUUCAGACCUGAAUGCCGCC AUGAAUAUUGCCGGGAUGCUGCUCACGCAGUGGCC UGGCAAUAGUGAGAAACGAUCUUCUUUUGGCAAGG CUAUCUCGUCCGAGAACCUUGUUUAUGAGAAGCCA CUUCCAACUGCGUAGCAGUUGGUGGUGGUCGUUC AC (SEQ ID NO:62) | 70% |

In one aspect of the present invention, the TnpB-like RNA-guided nuclease of the present invention is a protein comprising an amino acid sequence selected from the group consisting of the amino acid sequences of SEQ ID NOs: 2, 4, 6, 8, 10, 12, 14, 16, 18, 20, 22, 24, 26, 28, 30, 32, 34, 36, 38, 40, 42, 44, 46, 48, 49, 50, 52, 54, and 56 , or a mutant protein thereof.

The mutant protein refers to a protein that has an amino acid sequence having at least 50% sequence homology or identity to the amino acid sequence of the original protein and has TnpB-like RNA-guided nuclease activity. Such a mutant protein is characterized by comprising the following regular expression sequence in the RuvC domain: 
Y-K-G-R-T-F-[NS]-[KR]-[LM]-x-[AN]-x-G-[AS]-[KR]-[GS]-Q-Y-x(2)-R-[AS]-x-[DN]-
x-L-x-W-x-G
(SEQ ID NO:1)
 (wherein,
[NS] represents N or S;
[KR] represents K or R;
[LM] represents L or S;
x represents any amino acid;
[AN] represents A or N;
[AS] represents A or S;
[KR] represents K or R;
[GS] represents G or S;
x(2) represents any two amino acids;
[AS] represents A or S; and
[DN] represents D or N),
and has the size of the RuvC domain specified as being 200 to 350 residues, preferably 250 to 300 residues, in full length. Moreover, such a mutant protein has three-dimensional structural homology, and more preferably has structural homology in the RuvC domain.

The mutant protein may be specified by a homology and/or identity of 40% or more, 45% or more, 50% or more, 55% or more, 60% or more, 65% or more, 70% or more, 75% or more, 80% or more, 85% or more, 90% or more, 93% or more, 95% or more, 97% or more, 98% or more, or 99% or more relative to the amino acid sequence of the original protein.

In particular, it is particularly preferred that the mutant protein belongs to the same clade as the original protein. When it belongs to the same clade, the homology or identity is usually 50% or more, preferably 60% or more, 70% or more, or 80% or more.

The TnpB-like RNA-guided nuclease belonging to Clade 1 relates to:
(1) a protein consisting of an amino acid sequence selected from the group consisting of the amino acid sequences of SEQ ID NOs: 16, 20, 22, 24, 26, 32, 40, 42, 46, 50, 52, 54, and 56, particularly a protein consisting of an amino acid sequence selected from the group consisting of the amino acid sequences of SEQ ID NOs: 16, 20, 22, 24, 26, 40, 50, 52, 54, and 56; or
(2) a protein comprising an amino acid sequence having at least 50%, 60%, 70%, 75%, 80%, or 90% sequence homology or identity to an amino acid sequence selected from the group consisting of the amino acid sequences of SEQ ID NOs: 16, 20, 22, 24, 26, 32, 40, 42, 46, 50, 52, 54, and 56, particularly an amino acid sequence selected from the group consisting of the amino acid sequences of SEQ ID NOs: 16, 20, 22, 24, 26, 40, 50, 52, 54, and 56, wherein the sequence has TnpB-like RNA-guided nuclease activity.

The sequence identity can be appropriately selected so as not to include T11, T16, and T18.

The TnpB-like RNA-guided nuclease belonging to Clade 2 relates to:
(1) a protein consisting of an amino acid sequence selected from the group consisting of the amino acid sequences of SEQ ID NOs: 4, 6, 8, and 48 or
(2) a protein comprising an amino acid sequence having at least 40%, 50%, or 60% sequence homology or identity to an amino acid sequence selected from the group consisting of the amino acid sequences of SEQ ID NOs: 4, 6, 8, and 48, wherein the sequence has TnpB-like RNA-guided nuclease activity.

The TnpB-like RNA-guided nuclease belonging to Clade 3 relates to:
(1) a protein consisting of an amino acid sequence selected from the group consisting of the amino acid sequences of SEQ ID NOs: 10, 12, and 14; or
(2) a protein comprising an amino acid sequence having at least 80% sequence homology or identity to an amino acid sequence selected from the group consisting of the amino acid sequences of SEQ ID NOs: 10, 12, and 14, wherein the sequence has TnpB-like RNA-guided nuclease activity.

The TnpB-like RNA-guided nuclease belonging to Clade 4 relates to:
(1) a protein consisting of an amino acid sequence selected from the group consisting of the amino acid sequences of SEQ ID NOs: 2, 36, and 38; or
(2) a protein comprising an amino acid sequence having at least 60% sequence homology or identity to an amino acid sequence selected from the group consisting of the amino acid sequences of SEQ ID NOs: 2, 36, and 38, wherein the sequence has TnpB-like RNA-guided nuclease activity.

In one aspect, the TnpB-like RNA-guided nuclease of the present invention relates to:
(1) a protein encoded by a nucleic acid sequence selected from the group consisting of the nucleic acid sequences of SEQ ID NOs: 3, 5, 7, 9, 11, 13, 15, 17, 19, 21, 23, 25, 27, 29, 31, 33, 35, 37, 39, 41, 43, 45, 47, 51, 53, 55, and 57; or
(2) a protein comprising an amino acid sequence encoded by a nucleic acid sequence having at least 60% sequence identity to a nucleic acid sequence selected from the group consisting of the nucleic acid sequences of SEQ ID NOs: 3, 5, 7, 9, 11, 13, 15, 17, 19, 21, 23, 25, 27, 29, 31, 33, 35, 37, 39, 41, 43, 45, 47, 51, 53, 55, and 57, wherein the protein sequence has TnpB-like RNA-guided nuclease activity.

In the present specification, "homology" between two amino acid sequences refers to the ratio of identical or similar amino acid residues appearing at each corresponding position when the both amino acid sequences are aligned, and "identity" between two amino acid sequences refers to the ratio of identical amino acid residues appearing at each corresponding position when the both amino acid sequences are aligned. Note that "homology" and "identity" between two amino acid sequences can be determined using, for example, the BLAST (Basic Local Alignment Search Tool) program (Altschul et al., J. Mol. Biol., (1990), 215(3):403-10) or the like.

The mutant protein is a protein having structural homology to the RuvC domain of the original protein, and may be a protein further having structural homology relative to the full length of the original protein. Although the position of the RuvC domain varies depending on the type of T protein, as an example, in the case of T8-2 protein, R302 to E580 correspond to the RuvC domain. For other T proteins, the RuvC domain corresponding to such a position can be determined. Structural homology can be determined by an RMSD value and/or a TM-score when aligned with the three-dimensional structure of the original protein. The RMSD value refers to the root-mean-square deviation of atomic positions. As an example, when a Cα root-mean-square deviation (RMSD) between the backbone of the original protein and the backbone of the mutant is 2.5 Å or less, preferably 1.5 Å or less, and more preferably 1 Å or less, it can be said that there is a high probability of producing an effect equivalent to that exerted by the original protein. The RMSD value can be determined using software well known in the technical field, such as PyMOL software *align*. In addition to or instead of the RMSD value, a TM-score (Template Modeling score) can also be used. A protein having a TM-score of 0.9 or more, preferably 0.91 or more, and more preferably 0.93 or more can be referred to as a protein having structural homology. Therefore, the mutant can be specified by the RMSD value in addition to or instead of being specified by sequence identity or homology.

Specifically, the T8-2 protein belonging to Clade 1 and the TnpB-like RNA-guided nucleases belonging to Clades 1 to 4 have an RMSD value of 1.9 or less and a TM-score of 0.9 or more when structural alignment is performed using PyMOL software. In another example, in the case where they belong to the same clade, for instance, between the T8-2 protein belonging to Clade 1 and a TnpB-like RNA-guided nuclease belonging to Clade 1, the RMSD value is 1.5 or less and the TM-score is 0.91 or more when structural alignment is performed using PyMOL software. Since all proteins within the clade have TnpB-like RNA-guided nuclease activity, in the case where the RMSD between the original protein and the mutant protein is 1.9 or less, preferably 1.5 or less, and more preferably 1 or less, and the TM-score is 0.9 or more, preferably 0.91 or more, and more preferably 0.93 or more, it is inferred that the mutant protein has an activity equivalent to the original, and the mutant protein can be used for a genome editing tool by the procedure described in the present specification.

In another example, the three-dimensional structure and homology are compared between the T8-2 protein belonging to Clade 1 and a known high-activity TnpB RNA-guided nuclease. An example of the known high-activity TnpB RNA-guided nuclease includes DraTnpB-AI (SEQ ID NO: 174). By aligning the three-dimensional structures, amino acid positions contributing to the activity can be identified. In the amino acid sequence of the T8-2 protein (SEQ ID NO: 22), amino acid positions corresponding to Q284, R458, K89, S72 and/or S75, R291, S201, L278, and S315 can contribute to the activity of TnpB-like RNA-guided nucleases belonging to Clade 1. The corresponding amino acid positions can be determined by performing structural comparison or amino acid sequence comparison. In one aspect, the present invention comprises a sequence having a substitution at a corresponding position of at least one or a combination selected from the group consisting of Q284, R458, K89, S72 and/or S75, R291, S201, L278, and S315 in the amino acid sequence of SEQ ID NO: 22, in the amino acid sequence of a TnpB-like RNA-guided nuclease belonging to Clade 1. These mutations are particularly preferably a combination of two positions or a combination of three positions. As an example, a mutation in a combination of the positions of K89 and Q284, K89 and R291, Q284 and R291, or a combination of the positions of K89, Q284, and R291 is preferred.

Examples of such substitutions include: Q284R or Q284K at position 284 or a position corresponding thereto; K89R at position 89 or a position corresponding thereto; S72H, S72N, S72Q, S72K, S72R, S75H, S75N, S75Q, S75K, or S75R at position 72 or 75 or a position corresponding thereto; R291E or R291K at position 291 or a position corresponding thereto; Q201H or Q201S at position 201 or a position corresponding thereto; L278K or L278R at position 278 or a position corresponding thereto; and S315A or S135V at position 315 or a position corresponding thereto. These substitutions can enhance TnpB-like RNA-guided nuclease activity. More specifically, from the viewpoint of providing high genome editing efficiency, substitutions of K89R, Q284K, Q284R, R291K, or a combination thereof are preferred. As an example, K89R and Q284K, K89R and R291K, Q284K and R291K, or K89R, Q284K, and R291K are preferred (FIG. 12).

The TnpB-like RNA-guided nuclease of the present invention or a mutant protein thereof has TnpB-like RNA-guided nuclease activity in a wide temperature range, for example, 20 to 50°C, and particularly high activity at 30 to 45°C, and more preferably around 37°C (FIG. 8). As typical TnpB proteins and TnpB-derived proteins often exhibit the maximum activity around 40 to 50°C, the nuclease of the present invention can be used efficiently at a lower temperature. Since the activity is high not only around 37°C, which is the body temperature of homeothermic animals, but also at 25°C, it is particularly suitable for genome editing in living organisms such as plants, algae, mollusks, and microorganisms.

### [Guide RNA (gRNA)]

In the present invention, a guide RNA is designed based on a DNA sequence of a cleavage target and a TnpB-like RNA-guided nuclease to be used, and is configured to comprise a guide sequence and a gRNA scaffold sequence. Since the DNA of the cleavage target is cleaved in the vicinity of a TAM sequence, it is selected under the condition that it contains a TAM sequence. A sequence of 14 nt to 25 nt adjacent to the 3' side of the TAM sequence can be selected as a guide sequence. The guide sequence may match the DNA sequence of the cleavage target, or may contain one or several mismatch sequences therein. Even when the guide sequence does not completely match the target sequence, cleavage may occur due to an off-target effect. As for the gRNA scaffold, it is necessary to use a sequence that can be utilized by the TnpB-like RNA-guided nuclease to be used. Generally, the 3' end boundary of a locus (i.e., the boundary between the gRNA scaffold and the guide sequence) can be determined by sequence comparison in the locus of each TnpB-like RNA-guided nuclease. The four bases at the 3' end of the gRNA scaffold sequence can be defined as "Transposon End Motif (TEM)". The gRNA scaffold sequence may be 70 to 200 nt. The guide sequence may be designed to be adjacent to the 3' end of the scaffold sequence, i.e., the TEM sequence, or an arbitrary sequence of 20 to 100 nt may be inserted therebetween.

In TnpB-like RNA-guided nucleases within each clade, gRNA scaffold sequences have high homology/identity. As an example, within the same clade, the gRNA scaffold sequence can be used if it has an identity/homology of 70% or more, preferably 80% or more, and more preferably 90% or more. Representative gRNA scaffold sequences of each clade are as follows:

**[Table 7]**

| Clade | Sequence | Identity within Clade |
|---|---|---|
| Clade 1 (T8-2) | CCGAGAUGAUCGGGCGCUACCUGUUUCUCAGGCC CAAGAACGGAAUCCAACCAGCGGCCUUGGCCGCCU AGUGACAAACGUCUCCCCCCUGCCGACGGCCGUGG CCAUCGGUGGACGGGAAGCCCGGUCGUUUACGGC CGGGAGGAAGUCAC (SEQ ID NO:58) | 70% |
| Clade 2 (T3) | GGCGGACGGCACCCGGUGGACGCGGACCUGCAU GCCGCGGAGACGCUGGCGCGAUGGCUCUUCCUCA GGCCGAAACCGGCGGGUUCGCCCGCCUUGUGACA AAAACCUUCCGUCGGCGGAUGAGGCUCGAUGCCGC UUCCGCCGACAGGGAAGCCCCGGGUUUCAACCCG GGGAGGUUCAC (SEQ ID NO:59) | 70% |
| Clade 3 (T5) | CCUAAAGAAACAGUCAGUCAGGCCUUUGGUCUGGC UGCUGAGUGACAAAUGUUUCAAGUCGCCGCCAGAU GACUCAAUGCCAGCUUGCGGGAUGCCAGAAGCUC CUUCCUUCAGGUGGGAGUAGUUCAC (SEQ ID NO:60) | 70% |
| Clade 4 (T13) | GCGGCGCACACGCUGGCGCUGUGGCCUCUGCUGC GGCCGAAGAUGAACGACAGGACGGCGCUGGCCGC CUAGGGAUCCCUCGUGACAAAAGUCUUCCCGCGGC AGAGGGUGCGUCCCCGAGCCGCCGGGAAGCCCCG ACCACAGCGCAACGCGCUUGGUCGGGGAGGUUCA C (SEQ ID NO:61) | 70% |
| Clade 5 (T7) | GUGAAAAUCCCGGCAUUCAGACCUGAAUGCCGCC AUGAAUAUUGCCGGGAUGCUGCUCACGCAGUGGCC UGGCAAUAGUGAGAAACGAUCUUCUUUUGGCAAGG CUAUCUCGUCCGAGAACCUUGUUUAUGAGAAGCCA CUUCCAACUGCGUAGCAGUUGGUGGUGGUCGUUC AC (SEQ ID NO:62) | 70% |

The underline represents a TEM sequence.

Therefore, as the gRNA scaffold, the sequence of any one of SEQ ID NOs: 58 to 62, or a sequence having a sequence identity of 70% or more, preferably 80% or more, and more preferably 90% or more to said sequence can be used.

Based on the sequence identity within a clade, a more important region for activity can be identified for the gRNA scaffold sequence, and a shortened sequence comprising such an important region can also be prepared. Examples of the shortened sequences of the gRNA scaffold sequence of T8-2 include SEQ ID NO: 117 (gRNA1), SEQ ID NO: 118 (gRNA2), and SEQ ID NO: 119 (gRNA3). Alternatively, a shortened mutant sequence can also be selected that has at least 90%, more preferably at least 92%, at least 93%, 95%, at least 97%, at least 98%, or at least 99% sequence identity to SEQ ID NO: 119 (gRNA3) and can exert a function as an RNA scaffold.

A gRNA, which is designed based on a TnpB-like RNA-guided nuclease to be used and the nucleic acid sequence of a cleavage target can be expressed based on a technique well known in the technical field. As an example, a nucleic acid comprising an expression cassette in which a gRNA sequence is placed under the control of a promoter can be used. Such an expression cassette may comprise a transcription termination sequence in the 3' region. The nucleic acid comprising the expression cassette is mounted on a vector or a plasmid and is introduced *in vitro* or into a predetermined cell to generate a gRNA.

### [Inactivated-Mutation-Containing TnpB-like RNA-Guided Nuclease]

The TnpB-like RNA-guided nuclease can be inactivated by adding a substitution to an amino acid residue of its active domain. Examples of the active domain include D308, E489, and D575 in T8-2, and the TnpB-like RNA-guided nuclease can be inactivated by substituting a residue at these positions. While the inactivated TnpB-like RNA-guided nuclease is recruited to a target sequence by the action of a guide RNA, it does not exhibit RNA-guided nuclease activity. Further fusing of a functional domain to the inactivating-mutation-containing TnpB-like RNA-guided nucleasecan regulate a function for a gene in the target sequence. The functional domain to be fused may be any domain, and examples thereof include transcriptional regulatory domains such as p300, DMNT3, KRAB, SRDX, VPR, and VP64. In another example, any functional domain such as APOBEC, AID, TadA, Dda, FokI, M-MLV-RTase, or GFP can be utilized, which enables not only an epigenetic state but also modification to a base, reverse transcription, cleavage of a phosphate bond, and optical manipulation such as a fluorescent protein. An inactivating-mutation-containing RNA-guided nuclease fused with a functional domain is also widely known for CRISPR-Cas RNA-guided nucleases (Non-Patent Document 10: Int J Mol Sci., 2020 Apr 25;21(9):3038. doi: 10.3390/ijms21093038), and functional regulation is similarly possible for TnpB-like RNA-guided nucleases.

An inactivating-mutation-containing TnpB-like RNA-guided nuclease fused with a functional domain forms a complex with a guide RNA consisting of a sequence comprising a guide sequence and a gRNA scaffold sequence, and exerts a function corresponding to the functional domain. When a transcriptional activation domain, for example, all or part of the functional domain of p300, VPR, or VP64, is fused as the functional domain, transcription in the region recruited by the guide RNA is promoted. On the other hand, when a transcriptional repression domain, for example, all or part of the functional domain of DMNT3, KRAB, or SRDX, is fused as the functional domain, transcription in the region recruited by the guide RNA is repressed. Accordingly, the present invention may also relate to a method for regulating transcription of a target DNA using such an inactivating-mutation-containing TnpB-like RNA-guided nuclease fused with a functional domain.

### [Target-Specific Cleavage Method]

Another aspect of the present invention relates to a method for cleaving a target DNA using the TnpB-like RNA-guided nuclease complex according to the present invention. More specifically, the present invention relates to a method for cleaving a target DNA using a complex comprising:
(i) a TnpB-like RNA-guided nuclease according to the present invention; and
(ii) a guide RNA consisting of a sequence comprising a guide sequence and a gRNA scaffold sequence.

The complex may be formed *in vitro* and introduced into a cell using electroporation, liposomes, or the like to cleave the target DNA, or may cleave the target DNA *in vitro*. Alternatively,, the complex may be mounted in an expression cassette and introduced into a cell via gene introduction using a vector, messenger RNA, or the guide RNA itself, so that the TnpB-like RNA-guided nuclease and the guide RNA are expressed in the cell to cleave a genomic DNA. Such a cleavage method can be performed using a kit for cleaving target nucleic acids, which comprises the expression cassette.

Gene introduction can be performed using techniques known in the technical field. The method for introduction is not particularly limited and can be appropriately selected depending on the type of material to be introduced and the target of introduction. Methods for introduction are broadly classified into direct methods and methods using viral vectors. Examples of the direct methods include electroporation, a liposome method, a particle gun method in which shooting is performed together with gold particles, a whisker method, and the like. Examples of the methods using viral vectors include methods which use adenovirus, adeno-associated virus, lentivirus, *Agrobacterium*, tobacco mosaic virus (TMV)or the like as a vector depending on the host species.

Target-specific cleavage generated in a cell can be repaired by genomic DNA repair mechanisms. During that process, non-homologous end joining or homologous recombination occurs, thereby enabling knockout or knock-in of a gene. Therefore, the target-specific cleavage method of the present invention can also be referred to as a genome editing method. The genome editing method of the present invention may be performed on cultured cells as well as cultured tissues or cells of a living organism. Target species are not particularly limited, and genome editing is possible for any cells of bacteria, archaea, and eukaryotes. Eukaryotes may be any cells such as plant cells, insect cells, and animal cells. For example, genome editing is possible for any mammalian cells, and genome editing is possible for both human cells and non-human animal cells. The T protein according to the present invention is characterized by having a low optimal temperature. Therefore, it is also possible to perform genome editing at a temperature of 10 to 30°C.

### [Target-Specific Protein Recruitment]

Another aspect of the present invention relates to a method for recruiting a specific protein to a target DNA using the TnpB-like RNA-guided nuclease complex according to the present invention, wherein the complex has been engineered not to exert TnpB-like RNA-guided nuclease activity.

As the complex engineered not to exert TnpB-like RNA-guided nuclease activity, the activity of the TnpB-like RNA-guided nuclease may be inactivated, or a guide RNA comprising a sequence of a length that binds target-specifically while the TnpB-like protein does not exhibit DNA cleavage activity may be used by adjusting the length of the guide RNA. Such a guide RNA may be a guide sequence of a target sequence of about 10 bases. More specifically, the TnpB-like RNA-guided nuclease complex used in the method for recruiting a specific protein to target DNA comprises:
(i) the TnpB-like RNA-guided nuclease according to the present invention in which an amino acid residue of an active center is substituted with another amino acid;
(ii) a guide RNA consisting of a sequence or comprising a guide sequence and a gRNA scaffold sequence; and
(iii) a specific protein that binds to or interacts with the TnpB-like RNA-guided nuclease. By expressing or introducing such a complex into a cell, the complex is recruited near the site of the guide sequence without causing cleavage thereby enabling recruitment of a specific protein bound to the complex. Examples of the active center of the mutant TnpB-like RNA-guided nuclease described in (i) include a typical DED active center contained in the RuvC domain. In the case of T8-2, the activity of the TnpB-like guided nuclease can be inactivated by introducing mutations into D308, E489, and D575. Mutations can be appropriately selected to an extent that the activity can be inactivated, and an example thereof includes a mutation to alanine.

A binding site with another protein, such as an MS2 sequence, can be inserted into the RNA scaffold sequence of the guide RNA described in (ii). As the specific protein described in (iii), which is a protein that modifies DNA or an epigenetic state, specifically all proteins generally referred to as epigenetic factors can be used. More specifically, examples include p300, DMNT3, KRAB, SRDX, VPR, and VP64. In addition, proteins enabling base modification, reverse transcription, cleavage of phosphodiester bonds, and optical manipulation using fluorescent proteins may also be employed, such as fluorescent proteins (specifically, APOBEC, AID, TadA, Dda, FokI, M-MLV-RTase, GFP, etc., but not particularly limited thereto). Recruitment of such a specific protein to a target DNA region can alter the epigenetic state in the target DNA region.

### [Kit]

Another aspect of the present invention may relate to a kit for cleaving target nucleic acids in a cell or a kit for genome editing. The kit comprises:
(i) an expression cassette for the TnpB-like RNA-guided nuclease according to the present invention; and
(ii) an expression cassette for a guide RNA having a sequence comprising a gRNA scaffold sequence.

The expression cassette for a guide RNA is provided such that a guide sequence can be inserted therein. An expression cassette for a guide RNA can be prepared by designing a cleavage target sequence and introducing a guide sequence. As an example, the expression cassette for the TnpB-like RNA-guided nuclease and the expression cassette for the guide RNA into which the guide sequence has been introduced are incorporated into a plasmid, a vector, or the like and then introduced into a cell.

The expression cassette can be prepared by generally introducing a promoter sequence and placing a sequence encoding a TnpB-like RNA-guided nuclease or a guide RNA under the control of the promoter sequence. The promoter can control the expression of a downstream sequence transiently or constitutively. The expression cassette may be contained on a single polynucleotide or on different polynucleotides. The expression cassette may contain elements contributing to expression such as a terminator, a signal sequence, a tag sequence, a reporter sequence, a multiple cloning site or elements necessary for preparing a plasmid or a vector such as a drug resistance gene, an origin of replication, and the like as long as the activity of the TnpB-like RNA-guided nuclease is not impaired. From the viewpoint of ensuring that the expressed protein acts in the nucleus, it is desirable that a nuclear localization signal is added as the signal sequence. One or a plurality of nuclear localization signals may be arranged in series as the nuclear localization signal. The nuclear localization signal can be appropriately selected depending on the biological species. Accordingly, the TnpB-like RNA-guided nuclease expressed in the cell translocates into the nucleus and can cleave a target nucleic acid sequence in cooperation with a guide RNA in the nucleus.

The kit for genome editing according to the present invention may further appropriately contain other materials, reagents, instruments, and the like necessary for performing the genome editing method of the present invention, such as a nucleic acid introduction reagent and a buffer solution. Examples of the other materials necessary for performing the genome editing method of the present invention include a donor polynucleotide in addition to the expression cassette for the TnpB-like RNA-guided nuclease and/or the expression cassette for the guide RNA. Introduction of the donor polynucleotide into the nucleus makes it possible to knock in the donor polynucleotide into a cleavage site generated by the CRISPR/Cas system. The donor polynucleotide causes homologous recombination at the cleavage site by arranging homologous sequences to a sequence on the 5' side of the cleavage site and a sequence on the 3' side of the cleavage site at the 5' end and the 3' end of the introduction sequence, respectively.

All documents mentioned in the present specification are incorporated herein by reference in their entirety.

Hereinafter, the present invention will be described in more detail based on examples, but these examples are merely shown for convenience of explanation, and the present invention is not limited to these examples in any sense.

### EXAMPLES

### Example 1: Identification of TnpB-like RNA-Guided Nuclease Homologs

Homologs of TnpB-like RNA-guided nucleases were detected by NCBI BLASTP and TBLASTN against the NCBI non-redundant protein sequence database, using the amino acid sequence of the T8-2 protein (SEQ ID NO: 22) as a seed query. Similar searches were performed not only in NCBI but also in the MGnify database. Nucleotide sequences of the loci corresponding to each of the resulting TnpB-like RNA-guided nucleases were retrieved from the NCBI GenBank database. Based on phylogenetic tree analysis of the protein sequences by the neighbor-joining method, the TnpB-like RNA-guided nucleases were classified into five groups (Clades 1 to 5) (FIG. 1). The right-end boundary of the locus (i.e., the boundary between the guide RNA (gRNA) scaffold and the guide sequence) was determined by aligning the genomic sequences of the loci encoding the aforementioned T proteins using ClustalW and comparing the 3' ends. From the alignment, the guide-scaffold boundary was identified as the most downstream position where sequence conservation decreases sharply (FIG. 2). A sequence motif consisting of four nucleotides located at the 3' end of the scaffold was defined as a Transposon-Encoded Motif (TEM).

By aligning the amino acid sequences of TnpB-like RNA-guided nucleases included in Clades 1 to 4, a conserved sequence (regular expression) in these sequences was determined as follows: 
Y-K-G-R-T-F-[NS]-[KR]-[LM]-x-[AN]-x-G-[AS]-[KR]-[GS]-Q-Y-x(2)-R-[AS]-x-
[DN]-x-L-x-W-x-G
(SEQ ID NO:1)
 (wherein,
[NS] represents N or S,
[KR] represents K or R,
[LM] represents L or S,
x represents any amino acid,
[AN] represents A or N,
[AS] represents A or S,
[KR] represents K or R,
[GS] represents G or S,
x(2) represents any two amino acids,
[AS] represents A or S, and
[DN] represents D or N).

By performing a database search for this regular expression sequence using PHI-BLAST or the like, all T proteins belonging to Clades 1-4 can be specifically and comprehensively identified.

### Example 2: Construction of TnpB-like RNA-Guided Nuclease Expression Vectors

To produce recombinant TnpB-like RNA-guided nucleases in *E. coli* as fusion proteins with N-terminal 10× histidine-tagged maltose-binding protein-TEV protease cleavage site (His-MBP-TEV), a synthetic DNA fragment containing the His-MBP sequence was cloned between the *Nco*I and *Nde*I cleavage sites of pET28b using the NEBuilder HiFi DNA Assembly Kit (New England Biolabs). This was designated as the pAN36 vector. All genes encoding TnpB-like RNA-guided nucleases and the accompanying gRNA scaffolds were synthesized by Integrated DNA Technologies (IDT) (Table 8). For several synthetic TnpB-like RNA-guided nuclease genes, the 5' region of the coding sequence that does not overlap with the gRNA scaffold was codon-optimized for protein expression in *E. coli*. This DNA fragment was cloned into the pAN36 vector under the T7 promoter. In the resulting vector, the sequence encoding the TnpB-like RNA-guided nuclease was fused in-frame to the N-terminal His-MBP-TEV sequence. The expression vectors for His-MBP-TEV-TnpB-like RNA-guided nucleases are as described in Table 8.

**[Table 8]**

| Name | Sequence (after codon optimization) | Vector sequence |
|---|---|---|
| His-MBP-TEV-T2 | SEQ ID NO:63 | SEQ ID NO:64 |
| His-MBP-TEV-T3 | SEQ ID NO:65 | SEQ ID NO:66 |
| His-MBP-TEV-T4-1 | SEQ ID NO:67 | SEQ ID NO:68 |
| His-MBP-TEV-T5 | SEQ ID NO:69 | SEQ ID NO:70 |
| His-MBP-TEV-T6 | SEQ ID NO:71 | SEQ ID NO:72 |
| His-MBP-TEV-T7 | SEQ ID NO:73 | SEQ ID NO:74 |
| His-MBP-TEV-T8-1 | SEQ ID NO:75 | SEQ ID NO:76 |
| His-MBP-TEV-T8-2 | SEQ ID NO:77 | SEQ ID NO:78 |

### Example 3: Expression and Purification of TnpB-like RNA-Guided Nuclease-RNA Complexes in E. coli

For the expression of recombinant His-MBP-TEV fusion TnpB-like RNA-guided nucleases, *E. coli* Rosetta2 (DE3) pLysS strain (Novagen) was transformed with the His-MBP-TEV-fusion TnpB-like RNA-guided nuclease expression vector. The *E. coli* was cultured overnight at 37°C in Luria-Bertani (LB) medium supplemented with 50 µg/mL kanamycin and 34 µg/mL chloramphenicol. One liter of LB medium supplemented with 50 µg/mL kanamycin and 34 µg/mL chloramphenicol was inoculated with 10 mL of the overnight pre-culture and grown until the optical density (*OD₆₀₀*) reached 0.6. Subsequently, gene expression was induced by adding 0.25 mM IPTG, and the cells were grown at 18°C for 20 hours. The cells were harvested by centrifugation and stored at -70°C until use.

All subsequent purification steps were performed at 4°C. The cells were resuspended in Buffer A (50 mM Tris-HCl, pH 8.0, 500 mM NaCl, 5% (v/v) glycerol, and 25 mM imidazole) supplemented with bovine DNAse I (FUJIFILM Wako Pure Chemical), chicken lysozyme (FUJIFILM Wako Pure Chemical), and protease inhibitors (phenylmethylsulfonyl fluoride and Roche cOmplete ethylenediaminetetraacetic acid-free). After 30 minutes of incubation, the cells were disrupted by sonication (ULTRASONIC DISRUPTOR UD-211, TOMY) on ice. After removing cell debris by centrifugation at 40,000 g for 30 minutes, the supernatant was filtered through a 0.45 µm polyvinylidene fluoride (PVDF) membrane and bound to 1 mL of Ni-Sepharose 6 Fast Flow Resin (Cytiva) equilibrated with Buffer A for 1 hour by a batch method. This resin was packed into an Econo-Pac chromatography column (Bio-Rad), washed first with 14 mL of Buffer B (50 mM Tris-HCl, pH 8.0, 1 M NaCl, 5% (v/v) glycerol, 25 mM imidazole), and subsequently with 10 mL of Buffer A. Bound proteins were eluted with 3.5 mL of Buffer C (50 mM Tris-HCl, pH 8.0, 1 M NaCl, 5% (v/v) glycerol, 300 mM imidazole). Proteins in the eluate were separated by 5-20% (w/v) polyacrylamide gel SDS-PAGE, and the gel was stained with Coomassie Brilliant Blue (FIG. 3: SDS-PAGE gel). Peak fractions containing the fusion protein were transferred to nuclease-free tubes, flash-frozen in liquid nitrogen, and stored at -70°C until use. The resulting TnpB-like RNA-guided nuclease ribonucleoprotein (hereinafter referred to as RNP) samples were used for nucleic acid extraction and dsDNA cleavage analysis.

For *in vitro* double-stranded DNA cleavage analysis, TnpB-like RNA-guided nuclease RNP samples were further purified using a HiLoad 16/600 Superdex 200 pg column in Buffer D [25 mM Tris-HCl pH 8.0, 150 mM NaCl, 5 mM MgCl₂, 1% (v/v) glycerol, 1 mM DTT]. Pooled fractions were concentrated by ultrafiltration and further stored at -80°C until use.

### Example 4: Extraction and Analysis of gRNA Bound to TnpB-like RNA-Guided Nuclease

To extract RNA bound to the TnpB-like RNA-guided nuclease RNP, 180 microliters of the peak fraction containing RNP was vigorously mixed with 540 microliters of TRI Reagent (Molecular Research Center, Inc.) and 108 microliters of chloroform for 15 seconds, incubated at room temperature for 5 minutes, and then centrifuged at 12,000 g at 4°C for 15 minutes. The upper aqueous phase containing RNA extracted from the RNP was transferred to a new tube and mixed with 500 microliters of 100% (v/v) ethanol. The sample was applied to an RNA Clean & Concentrator-5 spin column (Zymo Research). After washing the spin column with a wash buffer, 13.6 units of DNAse (QIAGEN) were applied to the spin column and incubated at room temperature for 15 minutes to remove residual DNA. After washing the spin column three times with a buffer, the bound RNA was eluted with 15 microliters of nuclease-free water. Subsequently, 500 ng of the purified RNA was used for RNA library preparation. The RNA library was prepared using a SMARTer smRNA-Seq Kit for Illumina (TAKARA-BIO) according to the manufacturer's instructions. The resulting NGS adapter-ligated cDNA was amplified by 8 cycles of PCR using full-length Illumina NGS indexing primers. Amplified DNA fragments of 200 to 500 bp were size-selected by 3% (w/v) agarose gel electrophoresis and purified by gel extraction using a FastGene Gel/PCR Extraction Kit (FastGene). DNA fragments were pooled in equimolar ratios, and the concentration was adjusted to 50 pM using a Qubit dsDNA HS Assay Kit and a Qubit 3.0 Fluorometer (Thermo Fisher Scientific Inc.). The pooled library was mixed with 0.25 volumes of 50 pM PhiX control v3 (Illumina Inc.) and then used for 2 x 150 bp paired-end sequencing on an iSeq100 (Illumina Inc.).

The adapters were trimmed from the reads, and the reads were aligned to the template sequence using Bowtie2 software.

The identified sequence of the small RNA bound to each TnpB-like RNA-guided nuclease was defined as the sequence of the corresponding gRNA (FIG. 4, a figure merging CDS and NGS reads). Information regarding the gRNAs is summarized in the following table.

**[Table 9]**

| Name | gRNA sequence |
|---|---|
| T2 | CUGGCUCUUCCUACGACCUAAACCGGCGGCCCCGGCCGCCGCGCCGACCCUGUGACAA AAAACUUCAGCCGUGGGCGGGGGCUCAACGCCGCCGCUCACGGCAGGGAAGCCCCGG GGUUUAGCCCGGGGAGGUUCACCGUCCUGGUCGUCCUCGCCCUGAUCGC (SEQ ID NO:79) |
| T3 | AAGCUGGACUGGAGGGGAAUCGCGCAUUCUGCUGCCGAGCUAUUACACGAGCUCGA CCGACUGGCGGACCGGCACCGUGGACAAGACCCAGCGCAAGGACGCGUGGUUCACCGC GGCGGACGGCACCCGGUGGGACGCGGACCUGCAUGCCGCGGAGACGCUGGCGCGAUG GCUCUUCCUCAGGCCGAAACCGGCGGGUUCGCCCGCCUUGUGACAAAAACCUUCCGUC GGCGGAUGAGGCUCGAUGCCGCUUCCGCCGACAGGGAAGCCCCGGGUUUCAACCCGG GGAGGUUCACCCUCUACCUCGAUCCAACGGGCAUCGCUCAUGAGAGAUCCACCGCUUC GGGGCUUAUCCUCUGCAGGAAGGCAGG(SEQ ID NO:80) |
| T4-1 | GAAAUAUGACGCAAUGGUGGUGAGUGAGAAGCUCGAUUACGUCACCAUUCCCAUCGAC GAUGUCGGCUACAAAGGUCGGACGUUCAACCGCAUGAUCAACAAUGGCGCGCGAUCGC AGUAUACAUCUCGCGCUGACGACAAAUUGGCUUGGAAAGGACACCCGCAGAUCAAAAUA CCCAGCUUCUUCACCAGCAGCACGGACUGGCGUCACGGCCUUGUCGAUAAGGCACAGC GACGUGGACCACUCUUCAAGUCUGCAGCAGAUGGAAAGACCUGGGAUGCCGAUAUGCA CGGCGCUGAGAUGAUCGGGCGCUGGCUCGUUCUCAAGCCCAAAGAAACAGCCAGUCAG GCGGUAAGCCUGGCUGCUAAGUGACAAAUGUUUCAAAUCCCCGCCAGAUGGCUCAAUG CCAGCUGGCGGGGAUGGCAGAAGCUCCUUUCUUCAGGGAGGAGUAGUUCACCUGAAGG GCAGAAGCAUAUAU (SEQ ID NO:81) |
| T5 | CCGATATGCACGGCGCCGAAATGATCGGACGCTGGCTCTTCCTCAAGCCTAAAGAAACAG TCAGTCAGGCCTTTGGTCTGGCTGCTGAGUAGCAAAUGUUUCAAGUCGCCGCCAGAUGA CUCAAUGCCAGCUUGCGGGGAUGCCAGAAGCUCCUUCCUUCAGGUGGGAGUAGUUCAC GUUCUCCAGGCUGCUAUCCUUAGCAAAUUUAUACCC(SEQ ID NO:82) |
| T6 | GGAAUGGAACGGUGGACAAGGCGCAGCGGCGGAGCGGUGUUCAAGGCGGCCUUCG ACGGCCGCAAGUGGGACGCCGACCUGCAUGCGGCCGAGAUGAUCGGACGCUACCUGUU CCUCAGGCCGAAACAAGGAAUCCAAGUUGCGGCUUUGGCCGCCUAGUGACAAACGCCU UCCCCCUGCCGACGGCCGUGGCCAUCGGUGGACGGGAAGCCCGGUUCUUCAGGGUCG GGAGAAGUCACGCAUUCCCAACUGGUGAUGCCGCGAUCCGACAAUCAUGGGGACGAAA UCGAAAAG(SEQ ID NO:83) |
| T7 | ACUACAAGGGGCGGCGCUUCAACAAGAUGAUCAACAACGGGUCGAAGGGGCAAUACAU UCGCCGCGCAUCGGACAAGCUGCUCUGGAAUGGUAUUCCGGAGCUGGCAAUGCCCACC CCCUACACCUCCCAGGCCUGUCUCUACCACCAGCAGCUUGGGAAACGGCAGGGCGAAC GCUUCACCUGCCCCCGUUGUGAAAAAUCCCGGCAUUCAGACCUGAAUGCCGCCAUGAA UAUUGCCGGGAUGCUGCUCACGCAGUGGCCUGGCAAUAGUGAGAAACGAUCUUCUUUU GGCAAGGCUAUCUCGUCCGAGAACCUUGUUUAUGAGAAGCCACUUCCAACUGCGUAGC AGUUGGUGGUGGUCGUUCACUCGAGCUACUUAUAUUUGAUCUACAAUUUGUGC (SEQ ID NO:84) |
| T8-1 | UUCAAGGCGUCUUCCGACGGCCGCAAAUGGACGCCGACCUGCACGCUGCCGAGAUGA UCGGCCGCUACCUGUUCCUCAGGCCGAAACAGGAAAUCAAAGCGGAAGCCCUCGAGGC UGCCGCCUAGUGACAAACGCCUUCCCCCUGCCGGCGGCCGUGGCCAUCGGGGGACGG GAAGCCCGGUCCUUCAGGGCCGGGAGGAAGUCACGAAUUCGUAAUCAUGGUCAUAGCU G(SEQ ID NO:85) |

### Example 5: Construction of 7N-TAM Library Plasmid

The TAM sequence of the TnpB-like RNA-guided nuclease was determined using a plasmid library containing seven randomized nucleotides (7N). To prepare the 7N-TAM plasmid library, ssDNA containing seven randomized nucleotides (5'-AGCTATGACCATGATTACGAATTCNNNNNNNCTGCAGGAGCAAAGACC-3' (SEQ ID NO: 87)) was converted into dsDNA by reverse transcriptase (ReverTra Ace, Takara Bio) using a primer (5'-GTCTTTGCTCCTGCAG-3' (SEQ ID NO: 88)). This dsDNA fragment was assembled with pUC18 containing an NGS adapter sequence using an NEBuilder HiFi DNA Assembly Cloning Kit (NEB) to prepare a 7N-TAM plasmid library (Table 10, plasmid sequence). *E. coli* DH5alpha cells were transformed with the reaction mixture and cultured at 37°C on LB plates supplemented with 100 g/mL ampicillin. 100,000 or more colonies were washed from the plates, and the plasmid library was extracted using a NucleoBond Xtra Midiprep kit (Takara Bio).

**[Table 10]**

| Name | Plasmid sequence |
|---|---|
| 7N-TAM Plasmid Library | CGACGGCCAGTGCCAAGCTTGtcgtcggcagcgtcagatgtgtat aagagacagCATGCCTGCAGGTCGACTCGAGAGGATCGT TGGTCTTTGCTNNNNNNNCGAGCTCGAATTCGTAATCA TGGTCATAGCTGTTTCCTGTGTGAAATTGTTATCCGCTC ACAATTCCACACAACATACGAGctgtctcttatacacatctccgagc ccacgagacCCGGAAGCATAAAGT (SEQ ID NO:89) |
| Underlined text: pUC18 | |
| Lowercase text: NGS adapter sequences | |

### Example 6: Screening of TAM Sequences by In Vitro Transcription/Translation

An *in vitro* transcription/translation (IVTT) reaction was performed using a PUREfrex 2.0 reconstituted cell-free protein synthesis kit (GeneFrontier). According to the product instructions, DNA templates encoding a T7 promoter-driven TnpB-like RNA-guided nuclease and a T7 promoter-driven gRNA scaffold accompanied by a spacer sequence (20 nt: 5'-GAATTCGTAATCATGGTCAT-3' (SEQ ID NO: 90)) targeting the TAM library were prepared by PCR using the synthetic gene of the TnpB-like RNA-guided nuclease as a template. Illumina Nextera HT v2 dual index primers were used as PCR primers for DNA template preparation. The IVTT reaction was performed in 20 to 25 microliters of reaction solution using 100 ng of the TnpB-like RNA-guided nuclease template, 125 ng of the corresponding gRNA template having the spacer sequence, and 60 ng of the TAM library plasmid. An IVTT reaction containing no gRNA DNA template was used as a control. The reaction was incubated at 37°C for 4 hours and then quenched by adding 5 microliters of 100 mg/mL RNase A (Nippon Gene), followed by incubation at 37°C for 30 minutes. After adding 5 microliters of 1% SDS, the reaction was incubated at 50°C for 10 minutes to denature the protein. Four units of Proteinase K (FUJIFILM) were added, and the mixture was incubated at 37°C for 90 minutes to digest the protein. Undigested TAM library plasmids were recovered from the reaction mixture using a FastGene Gel/PCR Extraction Kit and used as a template for subsequent PCR. A DNA fragment of approximately 250 bp containing the TAM motif and the target sequence was PCR-amplified by 30 cycles using KOD One PCR Master Mix (Toyobo) and Illumina full-length Nextera NGS indexing primers. The amplified DNA fragments were separated by 2.5% (w/v) agarose gel electrophoresis and then purified by gel extraction. Preferentially depleted TAM motifs were identified by NGS amplicon sequencing of the TAM library plasmid as described above. TAM preferences were characterized from the FASTQ files using a custom Python script. Briefly, 7-nucleotide TAMs were extracted and counted. TAM frequencies were normalized to the sequencing depth of each sample. Sequence logos were created using WebLogo version 2.8.2 (http://weblogo.berkeley.edu/) using TAMs with at least a 10-fold depletion relative to the control (FIG. 5).

### Example 7: In Vitro dsDNA Cleavage Analysis

The 7N sequence was converted to a TAM sequence by site-directed mutagenesis to synthesize substrate plasmids containing the TAM sequence. Substrate plasmids containing different target sequences (5'-GTTCTCCAGGCTGCTATCCTTAGCA-3' (SEQ ID NO: 91), 5'-GAATTCGTAATCATGGTCATAGCTG-3' (SEQ ID NO: 92)) were synthesized by site-directed mutagenesis. A 716-bp substrate dsDNA was amplified by PCR using the TAM sequence plasmid and primer 1 (5'-AAAGGGGATGTGCTGCAAGG-3' (SEQ ID NO: 93)) and primer 2 (5'-TATCTTTATAGTCCTGTCGG-3' (SEQ ID NO: 94)). The amplified dsDNA fragment (20 nM) was mixed with purified TnpB-like RNA-guided nuclease RNP (1, 2, 4 uM) corresponding to the target sequence in 5 microliters of Buffer D and incubated for 2 hours. The reaction mixture was quenched by adding 2 microliters of Proteinase K (FUJIFILM Wako Pure Chemical) and incubated at 60°C for 5 minutes. The reaction products were analyzed using a MultiNA microchip electrophoresis system (SHIMADZU Inc.) (FIG. 6).

### Example 8: In Vitro Plasmid Cleavage Analysis

The 7N sequence was converted to a TAM sequence by site-directed mutagenesis to synthesize substrate plasmids containing the TAM. Specifically, substrate plasmids were constructed for T5 by adding 5'-TTCAT-3' or 5'-AACAT-3' and for T8-2 by adding 5'-TTAT-3' or 5'-AACAT-3', respectively to the 5' side of the target sequence 5'-GAATTCGTAATCATGGTCATAGCTG-3' (SEQ ID NO: 92). 50 ng of the substrate plasmid was mixed with purified TnpB-like RNA-guided nuclease RNP (4 uM) corresponding to the target sequence in 5 microliters of Buffer D and incubated at 37°C for 2 hours. The reaction mixture was quenched by adding 2 microliters of Proteinase K (FUJIFILM Wako Pure Chemical) and incubated at 60°C for 5 minutes. The reaction products were analyzed on a 1.0% (w/v) agarose gel (FIG. 7). As a result, a change in mobility due to cleavage was observed when the TAM sequence corresponded to the correct one.

### Example 9: In Vitro Plasmid Cleavage Analysis (Confirmation of Temperature Dependence)

50 ng of the substrate plasmid used in Example 8 was mixed with purified TnpB-like RNA-guided nuclease RNP (4 uM) corresponding to the target sequence in 5 microliters of Buffer D, and incubated for 2 hours for each condition at reaction temperatures varying from 20°C to 50°C at 5°C intervals. The reaction mixture was quenched by adding 2 microliters of Proteinase K (FUJIFILM Wako Pure Chemical) and incubated at 60°C for 5 minutes. The reaction products were analyzed on a 1.0% (w/v) agarose gel (FIG. 8). These results indicate that the active temperature of the TnpB-like RNA-guided nuclease used in the present invention is lower (around 37°C) compared to typical TnpB proteins and TnpB-derived genes whose activity reaches its maximum around 40 to 50°C.

### Example 10: Construction of T8 Protein and gRNA Expression Vectors

Plasmid vectors for expressing T8-1 protein or T8-2 protein and gRNA in human cultured cells were each constructed as follows.

The plasmid vector for expressing the T protein is a pUC-based transient expression plasmid vector (SEQ ID NO: 95)comprising a CAG promoter, a multiple cloning site (MCS) provided with SV40 NLS and Nucleoplasmin NLS on the C-terminal side of the T protein, and a bGH polyA signal. A human codon-optimized T8-1 CDS sequence (SEQ ID NO: 96, synthesized by IDT) or a human codon-optimized T8-2 sequence (SEQ ID NO: 97, synthesized by IDT) for the T protein was subcloned into the MCS, respectively.

The plasmid vector for expressing gRNA is a pUC-based transient expression plasmid vector (SEQ ID NO: 98) comprising a human U6 promoter, a non-coding RNA cloning site, and a hammerhead ribozyme sequence on the 3' side thereof. First, a gT8-1 gRNA scaffold sequence (SEQ ID NO: 99, synthesized by IDT) or a T8-2 gRNA scaffold sequence (SEQ ID NO: 100, synthesized by IDT) was introduced into the gRNA expression vector, respectively. Furthermore, six types of sequences targeting the *hAAVS1* gene (SEQ ID NOs: 101 to 106) were subcloned.

### Example 11: Evaluation of Genome Editing Efficiency Using HEK293FT

Solutions containing 100 ng of the two types of T8 protein expression vectors constructed in Example 10 (expressing T8-1 or T8-2) and 50 ng of the gRNA expression vectors (six types of gRNA expression vectors targeting the hAAVS1 gene, used one by one) were each mixed with Lipofectamine 3000 reagent and added to 96-well cultured HEK293FT cells (~10⁴ cells/well), and transfection was performed using all 12 combinations. As a comparative control, a condition in which a GFP protein expression plasmid (pEGFP-N1, Clontech) was introduced was prepared (referred to as Control).

After culturing for 3 days, DNA of the HEK293FT cells was extracted with an alkaline buffer (0.1 N NaOH) and crude-purified. Using 100 pg/µL of the DNA as a template, partial sequences of the hAAVS1 gene containing the genome editing target sequence were amplified by PCR using KOD One PCR Master Mix and a first-round PCR primer set (SEQ ID NO: 110 and SEQ ID NO: 111 when using any of gRNA1, gRNA2, or gRNA3; SEQ ID NO: 112 and SEQ ID NO: 113 when using any of gRNA4, gRNA5, or gRNA6) under the following conditions.

Similarly, using a 20-fold dilution of the first-round PCR solution as a template, a second-round PCR was performed under the following conditions using a second-round PCR primer set and second-round PCR primers containing Combinatorial Dual Index (CDI) sequences (5'-AATGATACGGCGACCACCGAGATCTACACNNNNNNNNTCGTCGGCAGCGTC-3' (SEQ ID NO: 107) and 5'-CAAGCAGAAGACGGCATACGAGATNNNNNNNNGTCTCGTGGGCTCGG-3' (SEQ ID NO: 108)), resulted in PCR amplification products provided with adapter sequences and index sequences at both ends for sequencing on an Illumina sequencer.

PCR conditions: 35 cycles of 98°C for 2 minutes, 98°C for 10 seconds, 55°C for 5 seconds, and 68°C for 3 seconds (common to the first and second rounds).

The resulting PCR products were subjected to agarose gel electrophoresis, and the target base sequence portion was cut out and purified. The concentration of the purified DNA was measured using a Qubit dsDNA HS Assay Kit and a Qubit 3.0 Fluorometer (Thermo Fisher Scientific, Waltham, MA, USA) and adjusted to 50 pM. The pooled library was mixed with 0.25 volumes of 50 pM PhiX control v3 (Illumina) and then used for 2 × 150-bp paired-end sequencing with an iSeq100 (Illumina, San Diego, CA, USA). The resulting Fastq files were analyzed with CRISPResso2 (Clement et al., 2019 Nature Biotechnology). In the case where the T8 protein exhibits a cleavage pattern similar to TnpB, insertions and deletions within 10 bases downstream of the target sequence are considered to be genome editing induced by the T8 protein. Therefore, the quantitative window width for mutations was set to 10 bp each from the expected cleavage site (FIG. 9 and FIG. 10). "Genome editing efficiency" was defined as a value representing the ratio of reads having an insertion or deletion within the quantitative window width among all sequenced reads.

FIG. 9 shows the results of evaluating the genome editing efficiency by the above procedure under conditions where each of gRNA1 to gRNA6 was co-expressed with T8-1 or T8-2, respectively. The vertical axis represents the genome editing efficiency (the ratio of reads with an insertion or deletion mutation in the target region among all reads), and the horizontal axis represents the conditions of the combination of transfected vectors. While the genome editing efficiency was almost 0 in the Control, both T8-1 and T8-2 exhibited the highest genome editing efficiency (approximately 0.006) when co-expressed with gRNA3. Next, gRNA5 was efficient (approximately 0.0015). Although T8-1 and T8-2 exhibited approximately the same level of genome editing efficiency, for gRNA1, gRNA2, and gRNA4, co-introduction with T8-2 resulted in higher genome editing efficiency compared to co-introduction with T8-1.

FIG. 10 shows specific examples of genome editing when the T8-2 protein expression vector and the gRNA3 expression vector were simultaneously introduced into HEK293FT cells and the genome sequence was analyzed. The target genome sequence is shown as "Reference," the site expected to be bound by gRNA is shown as "sgRNA" (rectangle), and the assumed cleavage site indicated by a dashed line. Among sequences output by the sequencer, those different from each other are aligned, and the number of reads (reads) and their percentage (%) relative to all reads are shown. Base substitutions are indicated in bold, and deletions are indicated by "-". When genome editing occurs, insertions or deletions are observed in the vicinity of the dashed line representing the expected cleavage site; multiple types of such reads (particularly deletions of 3 to 8 bases) were detected at a rate of about 0.1%. From the above, it was found that in HEK293FT cells into which the T8-2 expression vector and the gRNA3 expression vector were co-introduced, genome editing mainly consisting of deletions occurs in the target genome region.

### Example 12: Construction of Modified T8-2 Guide RNA Expression Vectors

Plasmid vectors for expressing T8-2 guide RNA in human cultured cells were constructed as follows.

A plasmid vector for expressing gRNA is a pUC-based transient expression plasmid vector (SEQ ID NO: 98) comprising a human U6 promoter, a non-coding RNA cloning site, and a hammerhead ribozyme sequence on the 3' side thereof. First, a T8-2 guide RNA scaffold sequence consisting of 152 nt (SEQ ID NO: 100, synthesized by IDT) was introduced into said gRNA expression vector. Furthermore, a sequence targeting the *hAAVS1* gene (SEQ ID NO: 103) was sub-cloned. A guide RNA composed of this T8-2 guide RNA scaffold sequence, the target sequence, and the hammerhead ribozyme sequence was designated as "gRNA original" (SEQ ID NO: 114).

Modified T8-2 guide RNA expression vectors were prepared as follows. A guide RNA expression vector (SEQ ID NO: 115) was prepared by removing only the hammerhead ribozyme sequence from the aforementioned transient expression plasmid vector (SEQ ID NO: 98). A guide RNA sequence composed only of the T8-2 guide RNA scaffold sequence (SEQ ID NO: 98) and the *hAAVS1* gene target sequence (SEQ ID NO: 100) were cloned into the cloning site of this vector. This guide RNA sequence was designated as "ddHDV" (SEQ ID NO: 116). Next, nucleotide sequences were stepwise deleted from the 5' side of the guide RNA scaffold sequence within the ddHDV sequence to create four types of shortened guide RNAs (synthesized by IDT) i.e.gRNA1 (guide RNA scaffold sequence length: 131 nt, SEQ ID NO: 117), gRNA2 (guide RNA scaffold sequence length: 111 nt, SEQ ID NO: 118), gRNA3 (RNA scaffold sequence length: 100 nt, SEQ ID NO: 119), and gRNA4 (guide RNA scaffold sequence length: 77 nt, SEQ ID NO: 120). By cloning these, a total of five types of modified T8-2 guide RNA expression vectors were prepared.

### Example 13: Evaluation of Genome Editing Efficiency by Modified T8-2 Guide RNA Using HEK293FT

Using the T8-2 protein expression vector constructed in Example 10 and the modified T8-2 guide RNA expression vectors constructed in Example 12, the effect of modifying the guide RNA scaffold sequence on genome editing efficiency was evaluated using the method of Example 11 (FIG. 11). As a comparative control, a condition in which a GFP protein expression plasmid (pEGFP-N1, Clontech) was introduced was prepared (referred to as "Control").

As a result of comparing the genome editing efficiency of the *hAAVS1* gene in experiments where the T8-2 protein expression vector and gRNA expression vectors having a common target sequence but are different in gRNA coding regions from each other were co-introduced, no insertion or deletion of bases into the *hAAVS1* gene was observed in the Control. When a guide RNA that is a gRNA original having a full-length 152 nt T8-2 guide RNA scaffold sequence or ddHDV excluding only the HDV sequence was co-expressed with T8-2, the genome editing efficiency was approximately 0.7% in each case. These results showed that removal of the hammerhead ribozyme sequence from the guide RNA does not affect genome editing efficiency. The genome editing efficiency when using gRNA1, in which the T8-2 guide RNA scaffold sequence was shortened to 131 nt, was also approximately 0.7%; however, when using gRNA2, in which the guide RNA scaffold sequence was shortened to 111 nt, the genome editing efficiency improved to approximately 1.1%, and for gRNA3, shortened to 100 nt, the genome editing efficiency improved to approximately 0.86%. On the other hand, in gRNA4, shortened to 77 nt, the genome editing activity was not observed. These results indicate that deletion of 21 nt from the 5' side of the original T8-2 guide RNA scaffold sequence improves the genome editing activity, and furthermore, that the RNA sequence contained in the gRNA scaffold of gRNA3 and gRNA4 plays an important role in maintaining the DNA cleavage activity of T8-2.

### Example 14: Construction of T8-2 Mutant Protein Expression Vectors

Similarly to Example 11, plasmid vectors expressing 24 types of human codon-optimized T8-2 mutant proteins (SEQ ID NOs: 121-168) were constructed in place of the T8-2 protein.

### Example 15: Evaluation of Genome Editing Efficiency by T8-2 Mutant Proteins Using HEK293FT

Similarly to Example 12, the genome editing efficiency of the T8-2 mutant proteins was evaluated. As a plasmid vector for expressing gRNA, a plasmid vector comprising SEQ ID NO: 118, which had the highest editing efficiency among the six types of gRNA vectors used in Example 13, was used (FIG. 12).

FIG. 12 shows the results of evaluating the genome editing efficiency by the above procedure under conditions where the T8-2 protein without mutations or each of the 24 types of T8-2 mutant proteins was simultaneously co-expressed with a gRNA3 expression vector. The vertical axis indicates the genome editing efficiency (ratio of reads having an insertion or deletion mutation in the quantitative target region among all reads), and the horizontal axis indicates the conditions of the transfected vectors. While the genome editing efficiency was almost 0 in the Control and approximately 0.3% when the T8-2 protein without mutations was expressed, the genome editing efficiency was approximately 1% in cases where the T8-2 mutant protein had the K89R mutation, the Q284K mutation, or the R291K mutation, respectively. Furthermore, in the case of T8-2 mutant proteins simultaneously having both the K89R mutation and the Q284K mutation, having both the K89R mutation and the R291K mutation, or having both the R291K mutation and the Q284K mutation, all exhibited a genome editing efficiency of approximately 2%. Furthermore, the T8-2 mutant protein having the K89R mutation, the R291K mutation, and the Q284K mutation simultaneously exhibited a genome editing efficiency of approximately 2.5%. Accordingly, it was concluded that the K89R mutation, the R291K mutation, and the Q284K mutation are mutations that each increase the genome editing efficiency of the T8-2 protein, and that a T8-2 mutant protein with increased genome editing can be obtained by a combination of these mutations.

### Example 16: Construction of a fusion protein expression plasmid composed of an inactivated T8-2 and a catalytic domain of an epigenetic factor histone acetyltransferase and a gRNA expression vector

In known TnpB, a typical DED active center sequence contained in the RuvC endonuclease domain is recognized as the active center. It is known that DNA cleavage activity is abolished by substituting an aspartic acid residue on the N-terminal side with alanine. Furthermore, it is known that expressing a fusion protein composed of a known inactivated Cas9 and a catalytic domain of histone acetyltransferase (p300 core), along with a guide RNA targeting a promoter sequence promotes acetylation of histone proteins present in the vicinity of the target sequence, and enables transcriptional activation of a nearby gene to be induced through a change in the epigenetic state of the promoter region. For the purpose of utilizing T8-2 in gene transcription activation technology, an artificial transcription activator was created, composed of an inactivated T8-2 created by amino acid substitution of the DED active center sequence and the p300 core.

Plasmid vectors for expressing a fusion protein composed of the inactivated T8-2 and the catalytic domain p300 core of histone acetyltransferase and a gRNA in human cultured cells were constructed as follows.

A human codon-optimized CDS sequence (SEQ ID NO: 170, synthesized by IDT) was prepared, encoding a fusion protein (dead T8-2-p300 core, SEQ ID NO: 169) comprising an inactivated T8-2 sequence in which the 308th aspartic acid residue constituting the DED active center sequence of T8-2 was substituted with alanine, a hemagglutinin tag at the C-terminal side of the inactivated T8-2, two SV40 NLSs, and a p300 core sequence of human-derived histone acetyltransferase. The fusion protein expression vector was prepared by cloning said CDS sequence between a CBh promoter and a bGH polyA signal of a transient expression plasmid vector (SEQ ID NO: 95).

In addition, a vector expressing the T8-2 guide RNA was prepared by sub-cloning a DNA sequence (SEQ ID NO: 171) targeting a Tet operator sequence (TetO) or a DNA sequence (SEQ ID NO: 172) targeting a sequence not present in the reporter vector or the human genome into the 3' side of the guide RNA scaffold sequence of the T8-2 guide RNA expression vector prepared in Example 10.

### Example 18: Construction of an eGFP expression reporter vector for evaluating transcriptional activation by dead T8-2-p300 core

An eGFP expression reporter vector for evaluating transcriptional activation by the dead T8-2-p300 core in human cultured cells was constructed as follows.

The reporter vector is a pUC-based transient expression plasmid vector (SEQ ID NO: 173, synthesized by VectorBuilder) comprising a TRE3G promoter having a repeat sequence composed of seven Tet operator sequences (TetO), and a Kozak sequence, an eGFP CDS sequence, and an SV40 polyA signal on the 3' side thereof.

### Example 19: Evaluation of transcriptional activation capability by dead T8-2-p300 core using HEK293FT

Transfection was performed by mixing 300 ng of the dead T8-2-p300 core expression vector constructed in Example 17 (Note: Source 432-433) and 300 ng of the T8-2 guide RNA expression vector (sgRNA (TetO target)) having the TetO sequence of the eGFP reporter vector as a target sequence or the guide RNA expression vector (sgRNA (No target)) having a sequence not present in the reporter vector or the human genome as a target sequence, along with 300 ng of the eGFP expression reporter vector prepared in Example 18, with Lipofectamine 3000 reagent, and adding the mixture to 96-well cultured HEK293FT cells (~10⁴ cells/well).

The transfected HEK293FT cells were cultured in Dulbecco's Modified Eagle Medium containing 10% fetal bovine serum under conditions of 37°C and 5% CO₂. After culture for 24 hours, the expression level of eGFP was evaluated by measuring the integrated value of fluorescence intensity generated from eGFP using a fluorescence microscope to assess the transcriptional activation capability (FIG. 13). The integrated value of eGFP fluorescence intensity of the cultured cells into which the dead T8-2-p300 core expression vector and gRNA (TetO) were introduced was approximately 7.1-fold higher than that of the cells into which the gRNA (No target) was introduced. These results indicate that the dead T8-2-p300 core fusion protein induces transcriptional activation of a gene located in the vicinity of the target sequence by changing the epigenetic state.

## Claims

1. A TnpB-like RNA-guided nuclease complex comprising:
(i) a TnpB-like RNA-guided nuclease that has a length of 300 to 700 amino acids and comprises the following sequence:
Y-K-G-R-T-F-N-K-M-I-N-N-G-S-K-G-Q-Y-N-x-R-S-x-N-x-L-K-W-R-G
(SEQ ID NO:109)
[wherein x represents any amino acid]; and
(ii) a guide RNA consisting of a sequence comprising a guide sequence and a gRNA scaffold sequence.

2. The TnpB-like RNA-guided nuclease complex according to claim 1, wherein the TnpB-like RNA-guided nuclease is a protein consisting of an amino acid sequence selected from the group consisting of SEQ ID NOs: 16, 20, 22, 24, 26, 40, 50, 52, 54, and 56.

3. The TnpB-like RNA-guided nuclease complex according to claim 1, wherein the TnpB-like RNA-guided nuclease is a mutant protein having a mutation accompanied by an amino acid substitution relative to the protein according to claim 2, and having RNA-guided nuclease activity.

4. The TnpB-like RNA-guided nuclease complex according to claim 3, wherein the mutant protein consists of an amino acid sequence having at least 75% sequence identity to the amino acid sequence of the TnpB-like RNA-guided nuclease complex according to claim 2.

5. The TnpB-like RNA-guided nuclease complex according to claim 3, wherein the mutant protein is a protein having structural homology to the original protein in a RuvC domain, wherein the structural homology is such that, when structural alignment between the original protein and the mutant protein is performed using PyMOL software, a C-alpha root-mean-square deviation (RMSD) between the backbone of the original protein and the backbone of the mutant protein is 1.5 or less and a TM-score is 0.9 or more.

6. The TnpB-like RNA-guided nuclease complex according to claim 1, wherein a TAM sequence of the TnpB-like RNA-guided nuclease is a sequence comprising TCAT.

7. The TnpB-like RNA-guided nuclease complex according to claim 6, wherein the TAM sequence comprises TTCAT.

8. The TnpB-like RNA-guided nuclease complex according to claim 1, wherein the TnpB-like RNA-guided nuclease is a TnpB-like RNA-guided nuclease consisting of the amino acid sequence of SEQ ID NO: 22 or a mutant protein thereof, and the gRNA scaffold sequence comprises the sequence of SEQ ID NO: 58 or a sequence having at least 70% identity to said sequence.

9. The TnpB-like RNA-guided nuclease complex according to claim 8, wherein the mutant protein has an amino acid substitution at least at one position selected from the group consisting of Q284, R458, K89, S72 and/or S75, R291, S201, L278, and S315.

10. The TnpB-like RNA-guided nuclease complex according to claim 9, wherein in the mutant protein:
when there is an amino acid substitution at position 284, the substitution is Q284R or Q284K;
when there is an amino acid substitution at position 89, the substitution is K89R;
when there is an amino acid substitution at position 72 or 75, the substitution is S72H, S72N, S72Q, S72K, S72R, S75H, S75N, S75Q, S75K, or S75R;
when there is an amino acid substitution at position 291, the substitution is R291E or R291K;
when there is an amino acid substitution at position 201, the substitution is Q201H or Q201S;
when there is an amino acid substitution at position 278, the substitution is L278K or L278R; and
when there is an amino acid substitution at position 315, the substitution is S315A or S135V.

11. The TnpB-like RNA-guided nuclease complex according to claim 1, wherein the TnpB-like RNA-guided nuclease is a TnpB-like RNA-guided nuclease consisting of the amino acid sequence of SEQ ID NO: 22 or a mutant protein thereof, and the gRNA scaffold sequence comprises a nucleic acid consisting of the nucleotide sequence of SEQ ID NO: 117 (gRNA1), SEQ ID NO: 118 (gRNA2), or SEQ ID NO: 119 (gRNA3), or a nucleotide sequence having 90% sequence identity thereto.

12. A method for cleaving a target DNA using the TnpB-like RNA-guided nuclease complex according to any one of claims 1 to 11.

13. The method for cleaving a target DNA according to claim 12, comprising introducing into a cell:
a nucleic acid comprising an expression cassette encoding the TnpB-like RNA-guided nuclease; and
a nucleic acid comprising an expression cassette encoding the guide RNA.

14. The method for cleaving a target DNA according to claim 12, wherein the method is used as a genome editing method comprising sequence-specific knockout and sequence-specific knock-in.

15. An inactivating-mutation-containing TnpB-like RNA-guided nuclease complex comprising:
(i) a TnpB-like RNA-guided nuclease having an inactivating mutation in the TnpB-like RNA-guided nuclease of the TnpB-like RNA-guided nuclease complex according to any one of claims 1 to 11, and fused with a transcriptional regulatory domain; and
(ii) a guide RNA comprising a guide sequence and a gRNA scaffold sequence.

16. A method for regulating transcription of a target DNA using the inactivating-mutation-containing TnpB-like RNA-guided nuclease complex according to claim 15.

17. The method for regulating transcription of a target DNA according to claim 15, comprising introducing into a cell:
an expression cassette encoding the inactivating-mutation-containing TnpB-like RNA-guided nuclease; and
an expression cassette encoding the guide RNA.

18. A kit for genome editing comprising the TnpB-like RNA-guided nuclease complex according to any one of claims 1 to 11.

19. The kit for genome editing according to claim 18, wherein the guide RNA comprising the gRNA scaffold sequence comprises a guide sequence designed from a cleavage target sequence.

20. The kit for genome editing according to claim 18, wherein a TAM sequence of the TnpB-like RNA-guided nuclease is a sequence comprising TCAT.

21. The kit for genome editing according to any one of claims 18 to 20, which is used for sequence-specific knockout and sequence-specific knock-in.
